# EUROPEAN PATENT APPLICATION

(11) **EP 4 806 335 A1**
(43) Date of publication of application: **16.09.2026**
(21) Application number: 26159948.4
(22) Date of filing: 20.02.2026
(51) Int. Cl.: A61B 1/00, A61B 5/107

(54) **MEDICAL SUPPORT DEVICE, MEDICAL SUPPORT METHOD, AND MEDICAL SUPPORT PROGRAM**

(30) Priority: 10.03.2025 JP 2025037778
(71) Applicant: FUJIFILM Corporation, Tokyo 106-8620 (JP)
(72) Inventor: MACHII, Yusuke, Tokyo, 106-8620 (JP)
(74) Representative: Dehns Germany Partnerschaft mbB

(57) **Abstract**

Provided are a medical support device, a medical support method, and a medical support program capable of supporting understanding a spatial configuration of a surgical field.

A medical support device (11) includes a processor (41), in which the processor (41) is configured to acquire a surgical field image (21) obtained by capturing a surgical field (SF) with a camera (13B), generate a relative depth map (90) in which a relative depth is assigned for each region of the surgical field image (21), based on the surgical field image (21), estimate a marker depth that is an absolute depth of a marker (M) shown in the surgical field image (21), based on the surgical field image (21), and convert at least a part of the relative depths included in the relative depth map (90) into an absolute depth based on the marker depth.

## Description

### Technical Field

The present disclosure relates to a medical support device, a medical support method, and a medical support program.

### Background Art

In recent years, minimally invasive surgery using an endoscope (hereinafter referred to as "endoscopic surgery") has become widespread, and is expected to reduce a burden associated with surgery and promote postoperative recovery. In order to perform accurate operations in endoscopic surgery, it is essential to understand a spatial configuration within a surgical field, including a distance between a treatment tool and an organ. Further, in a case of constructing various applications that assist endoscopic surgery, it is important to understand three-dimensional information such as a depth and a shape of the surgical field.

Examples of a method of estimating three-dimensional information in an image in a general computer vision field include a depth estimation technology. The depth estimation technology is roughly classified into a technology of estimating a relative depth indicating a relative value corresponding to a distance from a camera and a technology of estimating an absolute depth indicating a physical distance (for example, in units of cm) from a camera.

The absolute depth is estimated by a machine learning model that has been trained using a combination of depth information as an absolute value acquired by a sensor such as light detection and ranging (LiDAR) and an image. In addition, for example, JP2024-524081A discloses generating a depth map corresponding to an endoscopic image based on a stereoscopic disparity or based on another depth detection technology (for example, a device using time-of-flight).

In endoscopic surgery, since surgery is performed in a narrow and complicated environment, it is not easy to acquire an accurate absolute depth (measurement value) using LiDAR, stereoscopic disparity, or the like. Therefore, in recent years, a method using a small amount of intraoperative data and a relative depth estimation model has been increasingly utilized in the field of endoscopic surgery. For example, in "Wei, R., Li, B., Chen, K., Ma, Y., Liu, Y., Dou, Q., "Enhanced Scale-aware Depth Estimation for Monocular Endoscopic Scenes with Geometric Modeling," Medical Image Computing and Computer-Assisted Intervention - MICCAI 2024, Lecture Notes in Computer Science, vol 15006. Springer.", a method of performing scale-correction on a relative depth map generated based on a monocular endoscopic image by using geometric modeling of a treatment tool is proposed.

### SUMMARY

During surgery, a treatment tool and/or a camera are moved, so that the entire treatment tool is not always included in the field of view of a surgical field image. In addition, in a case where a position or an orientation of the treatment tool is changed or a plurality of treatment tools are used in combination, it is difficult to identify the treatment tool used for depth estimation in the surgical field image. Therefore, in the related art method using the geometric modeling of the treatment tool, there is a case where real-time performance and accuracy are insufficient.

The present disclosure provides a medical support device, a medical support method, and a medical support program capable of supporting understanding of a spatial configuration of a surgical field.

According to a first aspect of the present disclosure, there is provided a medical support device comprising: a processor, in which the processor is configured to acquire a surgical field image obtained by capturing a surgical field with a camera, generate a relative depth map in which a relative depth is assigned for each region of the surgical field image, based on the surgical field image, estimate a marker depth that is an absolute depth of a marker shown in the surgical field image, based on the surgical field image, and convert at least a part of the relative depths included in the relative depth map into an absolute depth based on the marker depth.

The processor may be configured to generate an absolute depth map in which an absolute depth is assigned for each region of the surgical field image, based on the relative depth map and the marker depth.

The processor may be configured to perform control of displaying the surgical field image and the absolute depth map side by side or in a superimposed manner.

The surgical field may include a plurality of the markers, and the processor may be configured to estimate the marker depth for each marker based on the surgical field image, and convert at least a part of the relative depths included in the relative depth map into an absolute depth based on a plurality of the marker depths.

The processor may be configured to estimate validity of each marker depth, and convert at least a part of the relative depths included in the relative depth map into an absolute depth based on a plurality of the marker depths and the validity.

The marker may be assigned to a medical instrument, and the processor may be configured to acquire dimension information for each medical instrument, and estimate the marker depth based on the surgical field image and the dimension information.

The marker may be assigned to a medical instrument, and the processor may be configured to acquire presence information regarding a region in which each medical instrument is present in the surgical field image, and estimate the marker depth based on the surgical field image and the presence information.

The processor may be configured to acquire a plurality of the surgical field images that are temporally different, for each of the surgical field images, generate the relative depth map, estimate the marker depth, and perform conversion into the absolute depth, calculate an amount of variation in a corresponding absolute depth across the plurality of surgical field images, and derive a single absolute depth based on the absolute depth converted for each of the surgical field images in a case where the amount of variation is equal to or less than a predetermined threshold value.

The processor may be configured to estimate validity of the absolute depth converted for each of the surgical field images, and derive a single absolute depth based on the absolute depth converted for each of the surgical field images and the validity in a case where the amount of variation is equal to or less than the predetermined threshold value.

The processor may be configured to extract a landmark from the surgical field image, derive a landmark depth by converting a relative depth of a region that is included in the relative depth map and that corresponds to the landmark into an absolute depth based on the marker depth, and convert a relative depth of at least a part of regions other than the region that is included in the relative depth map and that corresponds to the landmark into an absolute depth based on the landmark depth.

The processor may be configured to convert a relative depth of at least a part of regions other than the region that is included in the relative depth map and that corresponds to the landmark into an absolute depth based on the landmark depth in a case where the marker depth is not able to be estimated.

The processor may be configured to estimate at least two marker depths based on the surgical field image, derive scale information of a space shown in the surgical field image based on the at least two marker depths, and perform control of displaying information corresponding to the scale information.

The processor may be configured to extract a superimposition object from the surgical field image, and perform control of superimposing and displaying gradations related to the superimposition object on the surgical field image based on the absolute depth and the scale information.

The processor may be configured to acquire a three-dimensional image of the surgical field, extract a region of interest from the surgical field image, identify a corresponding region that corresponds to a field of view of the surgical field image in the three-dimensional image based on the absolute depth and the region of interest, and perform control of displaying the three-dimensional image based on the corresponding region.

The processor may be configured to acquire a three-dimensional image of the surgical field, extract a region of interest from the surgical field image, register the region of interest with the three-dimensional image based on the absolute depth and the region of interest, and perform control of superimposing and displaying the three-dimensional image on the surgical field image based on a result of the registration.

The processor may be configured to identify at least one of a corresponding region that corresponds to a field of view of the surgical field image in the three-dimensional image or a non-rigid deformation parameter based on the result of the registration, and perform control of superimposing and displaying the three-dimensional image on the surgical field image based on a result of the identification.

The processor may be configured to acquire a three-dimensional image of the surgical field, identify an adjacent blood vessel closest to a viewpoint of the surgical field image based on the surgical field image and the three-dimensional image, and derive a distance between the adjacent blood vessel and a predetermined position based on the surgical field image and the absolute depth.

The processor may be configured to acquire a three-dimensional image of the surgical field, identify a target part inside an organ based on the three-dimensional image, and derive a distance between the target part and a predetermined position based on the three-dimensional image, the surgical field image, and the absolute depth.

According to a second aspect of the present disclosure, there is provided a medical support method executed by a computer, the medical support method comprising: acquiring a surgical field image obtained by capturing a surgical field with a camera; generating a relative depth map in which a relative depth is assigned for each region of the surgical field image, based on the surgical field image; estimating a marker depth that is an absolute depth of a marker shown in the surgical field image, based on the surgical field image; and converting at least a part of the relative depths included in the relative depth map into an absolute depth based on the marker depth.

According to a third aspect of the present disclosure, there is provided a medical support program causing a computer to execute: acquiring a surgical field image obtained by capturing a surgical field with a camera; generating a relative depth map in which a relative depth is assigned for each region of the surgical field image, based on the surgical field image; estimating a marker depth that is an absolute depth of a marker shown in the surgical field image, based on the surgical field image; and converting at least a part of the relative depths included in the relative depth map into an absolute depth based on the marker depth.

According to the above aspects, a medical support device, a medical support method, and a medical support program of the present disclosure can support understanding of a spatial configuration of a surgical field.

### BRIEF DESCRIPTION OF DRAWINGS

FIG. 1 is a diagram showing an outline of a medical support system including a medical support device.
FIG. 2 is a diagram showing a state of an inside of a body in an endoscopic surgery.
FIG. 3 is a diagram showing an example of a hardware configuration of the medical support device.
FIG. 4 is a diagram showing an example of a functional configuration of the medical support device according to a first embodiment.
FIG. 5 is a diagram showing an example of a relative depth map.
FIG. 6 is a diagram showing a relationship in a position and an orientation between a marker and an ultrasound probe.
FIG. 7 is a diagram showing an example of a surgical field image.
FIG. 8 is a diagram showing a relationship in a position and an orientation between a marker and an ultrasound probe.
FIG. 9 is a diagram showing an example of a surgical field image.
FIG. 10 is a diagram showing an example of an absolute depth map.
FIG. 11 is a flowchart showing an example of medical support processing according to the first embodiment.
FIG. 12 is a diagram showing an example of a functional configuration of the medical support device according to a second embodiment.
FIG. 13 is a diagram showing an example of a surgical field image.
FIG. 14 is a diagram showing an example of gradations.
FIG. 15 is a diagram showing an example of gradations.
FIG. 16 is a diagram showing an example of a puncture path and gradations.
FIG. 17 is a diagram showing an example of a three-dimensional image.
FIG. 18 is a diagram showing an example of a display aspect of a three-dimensional image.
FIG. 19 is a diagram showing an example of a display aspect of a three-dimensional image.
FIG. 20 is a diagram showing an example of a display aspect of a three-dimensional image.
FIG. 21 is a diagram showing an example of a display aspect of a three-dimensional image.
FIG. 22 is a diagram showing an example of a display aspect of a three-dimensional image.

### DESCRIPTION OF EMBODIMENTS

Hereinafter, an example of an embodiment of the disclosed technology will be described with reference to the drawings. The same or equivalent components and parts in the respective drawings are denoted by the same reference numerals, and the duplicated description will be omitted. In addition, dimensional ratios in the drawings are exaggerated for convenience of description and may be different from the actual ratios.

### First Embodiment

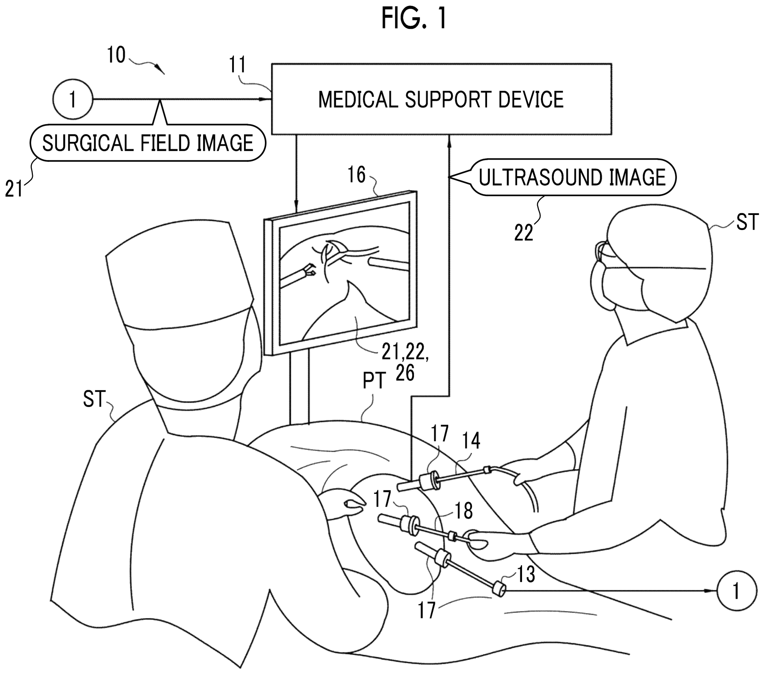

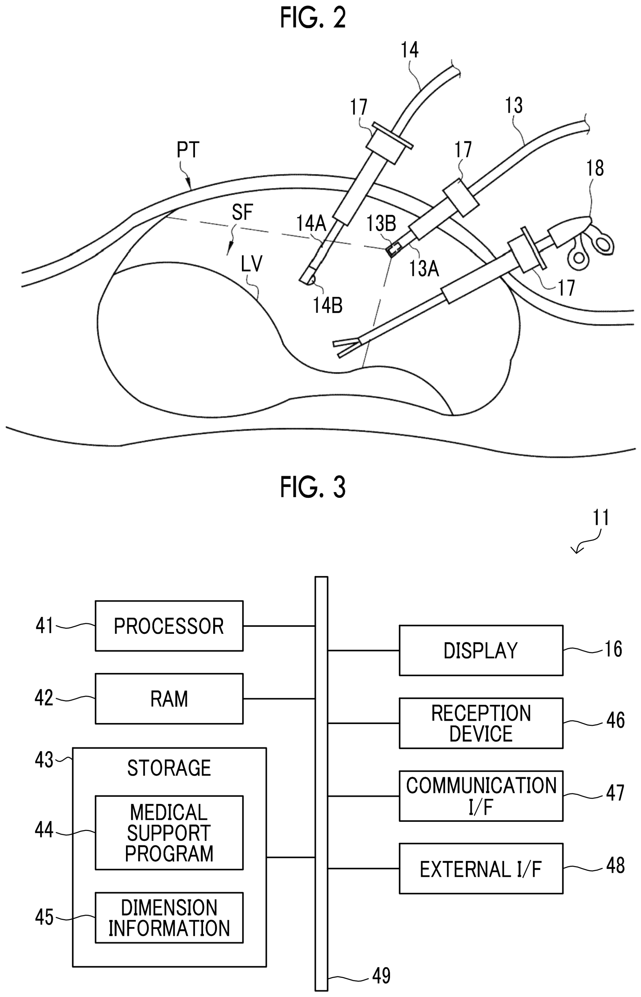

An example of a medical support system 10 to which a medical support device 11 according to the present embodiment is applied will be described with reference to FIGS. 1 and 2. As an example, the medical support system 10 is used in a case where an endoscopic surgery using an endoscope 13 is performed on a patient PT. The endoscopic surgery is a surgery that is performed by making a small hole in a body of the patient PT and inserting a medical instrument such as the endoscope 13 through the hole, unlike a laparotomy.

The medical support system 10 provides a medical staff ST including a doctor with a view of a surgical field inside the body of the patient PT, and with support information for supporting medical care such as a surgery and an examination. The support information is an absolute depth map 92 and the like as will be described below. Such a medical support system 10 has a function of providing the support information in real time during a surgery, and is therefore also called a surgical navigation system or the like.

As shown in FIG. 1, the medical support system 10 comprises a medical support device 11, an endoscope 13, an ultrasound probe 14, and a display 16. The medical support device 11 is communicably connected to the endoscope 13, the ultrasound probe 14, and the display 16.

FIG. 2 shows a state in which the endoscope 13 and the ultrasound probe 14 are inserted into the abdomen of the patient PT. In the endoscopic surgery, a part of the endoscope 13 and a part of the ultrasound probe 14 including distal end parts thereof are inserted into the body via a trocar 17. The trocar 17 is an insertion tool having an insertion hole into which the endoscope 13 or the like is inserted and a valve provided in the insertion hole to prevent gas leakage. In the endoscopic surgery, the trocar 17 is used for insertion into the body, such as the endoscope 13 and the ultrasound probe 14, because pneumoperitoneum is performed by injecting carbon dioxide gas into an abdominal cavity.

A treatment tool 18 is, for example, forceps, and the treatment tool 18 is also inserted into the body via the trocar 17. The treatment tool 18 is not limited to the forceps, and may be, for example, a biopsy needle, a tissue sampling needle, an injection needle, a snare, an electric scalpel, or a high-frequency knife. At least one of the ultrasound probe 14 or the treatment tool 18 is an example of a "medical instrument" of the present disclosure.

The endoscope 13 optically images a surgical field SF including a target part (in this example, the liver LV) inside the body of the patient PT by using a camera 13B. The surgical field SF is a space that spreads in a body cavity defined by an organ and a body wall inside the body. Specifically, the endoscope 13 has an insertion part 13A to be inserted into the body of the patient PT. The camera 13B and a light source (for example, a light emitting diode (LED)) for illumination are incorporated in a distal end part of the insertion part 13A. The endoscope 13 is, for example, a rigid endoscope in which the insertion part 13A is rigid, and is often used for abdominal cavity observation, so that the endoscope 13 is also called a laparoscope.

The camera 13B has an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The endoscope 13 is connected to an image processing processor for an endoscope (not shown). This image processing processor performs signal processing on an imaging signal output by the image sensor to generate a surgical field image 21 of the surgical field SF inside the body. The surgical field image 21 captured by the endoscope 13 is transmitted to the medical support device 11 in real time via the image processing processor for an endoscope. The camera 13B is an example of a "camera" of the present disclosure.

As illumination light for the endoscope 13, for example, visible light such as white light is used. As the illumination light of the endoscope 13, special light such as ultraviolet light and infrared light may be used. As the special light, for example, light restricted to a specific wavelength such as short-wavelength narrow-band light obtained by narrowing down light in a short wavelength range such as an ultraviolet range may be used. The surgical field image 21 is a video of the surgical field SF illuminated with illumination light, and more specifically, is a video based on light reflected from the illumination light near the surface of the surgical field SF. Therefore, in the surgical field image 21, a structure present in the vicinity of a surface layer of the target part can be visualized, but it is difficult to observe an internal structure.

The ultrasound probe 14 transmits an ultrasound wave to the target part and detects an electric signal corresponding to an ultrasound echo reflected from the target part. Specifically, the ultrasound probe 14 has an insertion part 14A that is inserted into the body of the patient PT. An ultrasound transducer 14B is incorporated in a distal end part of the insertion part 14A.

The ultrasound transducer 14B transmits an ultrasound wave to the target part and receives an ultrasound echo reflected from the target part. The ultrasound probe 14 is connected to an image processing processor for an ultrasound probe (not shown). This image processing processor performs image reconstruction processing based on an electric signal corresponding to the ultrasound echo received by the ultrasound transducer 14B so that an ultrasound image corresponding to the electric signal is generated. Through the image reconstruction processing, the ultrasound image 22 showing an internal structure of the target part scanned by the ultrasound probe 14 is generated. The ultrasound image 22 captured by the ultrasound probe 14 is transmitted to the medical support device 11 in real time via the image processing processor for an ultrasound probe.

The ultrasound probe 14 is, for example, a convex type that radially transmits ultrasound waves, and acquires a fan-shaped ultrasound image 22 with the ultrasound transducer 14B as a base point. A plurality of the ultrasound images 22 are captured along a scanning direction by performing the scanning with the ultrasound probe 14. The ultrasound image 22 is a so-called brightness (B)-mode image in which an internal structure from a surface layer to a deep layer where the ultrasound wave reaches the target part is visualized as brightness information. The ultrasound image 22 visualizes an internal structure of the target part that cannot be observed in the surgical field image 21 obtained by optical imaging.

The medical support device 11 acquires the surgical field image 21 from the endoscope 13, and acquires the ultrasound image 22 from the ultrasound probe 14. The medical support device 11 performs control of displaying the surgical field image 21 and/or the ultrasound image 22 on the display 16. A field of view inside the body of the patient PT is provided to the medical staff ST through a screen of the display 16.

In addition, the medical support device 11 provides support information for supporting understanding of a spatial configuration of the surgical field SF. Specifically, the medical support device 11 estimates a depth of a space shown in the surgical field image 21 as the support information. The term "depth" refers to a distance in a depth direction from a viewpoint of the camera. In a general computer vision field, a relative depth indicating a relative value corresponding to a distance from a camera and an absolute depth indicating a physical distance (for example, in units of cm) from a camera are used as depth information. The medical support device 11 estimates the absolute depth of the surgical field SF.

Hereinafter, a detailed configuration of the medical support device 11 will be described. FIG. 3 shows an example of a hardware configuration of the medical support device 11. The medical support device 11 comprises a display 16, a processor 41, a random access memory (RAM) 42, a storage 43, a reception device 46, a communication interface (I/F) 47, and an external I/F 48. These units are connected to a bus 49 such as a system bus and a control bus, and can communicate with each other.

In addition to the surgical field image 21, the ultrasound image 22, and the support information, various types of information are displayed on the display 16. Examples of the display 16 include a liquid crystal display and an electro-luminescence (EL) display. The number of the displays 16 need only be at least one as shown in FIG. 1, and may be more than one.

The processor 41 is, for example, a central processing unit (CPU), and integrally controls the respective units of the medical support device 11 in accordance with a control program and executes various types of processing in accordance with various types of application programs. The processor 41 is an example of a "processor" of the present disclosure.

The RAM 42 is a memory that transitorily stores information, and is used as a work memory by the processor 41. Examples of the RAM 42 include a dynamic random access memory (DRAM) and a static random access memory (SRAM).

The storage 43 is a non-volatile storage device that stores various programs, various parameters, and the like. Examples of the storage 43 include a hard disk drive (HDD) and a solid state drive (SSD). The storage 43 stores a medical support program 44 for causing a computer to function as the medical support device 11.

In addition, the storage 43 stores dimension information 45. The dimension information 45 includes information about a geometric size and shape of a medical instrument (the ultrasound probe 14, the treatment tool 18, and the like) to which a marker M described below is assigned. This is, for example, information indicating a length, a width, a diameter, a height, a thickness, and a structural feature (linear or curved shape) of the entire medical instrument and/or a specific part (such as distal end part) of the medical instrument. In addition, the dimension information 45 includes information about a shape of the marker M and placement of the marker M on the medical instrument. This is, for example, information indicating how the marker M is provided in an axial direction and a circumferential direction of the medical instrument.

The reception device 46 includes a keyboard, a mouse, and the like (not shown), and receives an instruction from the operator. That is, the medical support device 11 is operated by an operator such as the medical staff ST through the reception device 46. The reception device 46 may be a device that receives touch input, such as a touch panel, a device that receives voice input, such as a microphone, a device that receives gesture input, such as a camera, or the like.

The communication I/F 47 is connected to a network (not shown) such as a local area network (LAN) and/or a wide area network (WAN), and performs transmission control in accordance with a communication protocol defined in various types of wired or wireless communication standards. The external I/F 48 is, for example, a universal serial bus (USB) interface, and is used for connection to peripheral devices such as a printer and a memory card. As the medical support device 11, for example, a personal computer, a server computer, a smartphone, a tablet terminal, or a wearable terminal can be applied as appropriate.

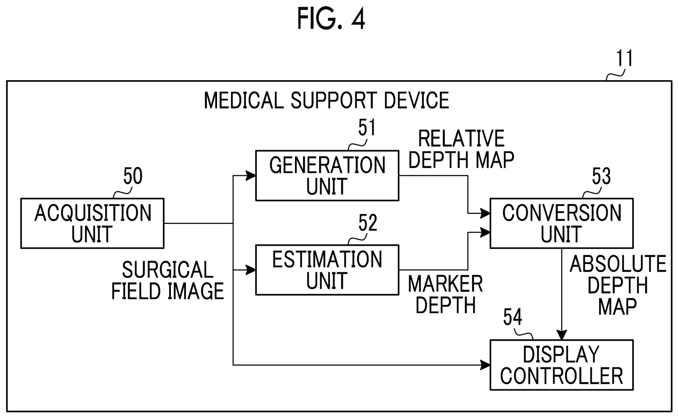

Next, a functional configuration of the medical support device 11 will be described. FIG. 4 is a block diagram showing an example of the functional configuration of the medical support device 11. The medical support device 11 comprises an acquisition unit 50, a generation unit 51, an estimation unit 52, a conversion unit 53, and a display controller 54 as functional units. The processor 41 reads out the medical support program 44 from the storage 43 and executes the medical support program 44 on the RAM 42, so that the processor 41 functions as each functional unit. The processor 41 operates as each functional unit to implement the medical support processing.

The acquisition unit 50 acquires the surgical field image 21 obtained by optically capturing the surgical field SF with the camera 13B. For example, the acquisition unit 50 acquires the surgical field image 21 from a device including a processor of the endoscope 13 via the external I/F 48 or the communication I/F 47. The processor of the endoscope 13 may be provided in the medical support device 11.

In addition, the acquisition unit 50 acquires the ultrasound image 22 captured by the ultrasound probe 14. For example, the acquisition unit 50 acquires the ultrasound image 22 from the device including the processor of the ultrasound probe 14 via the external I/F 48 or the communication I/F 47. The processor of the ultrasound probe 14 may be provided in the medical support device 11.

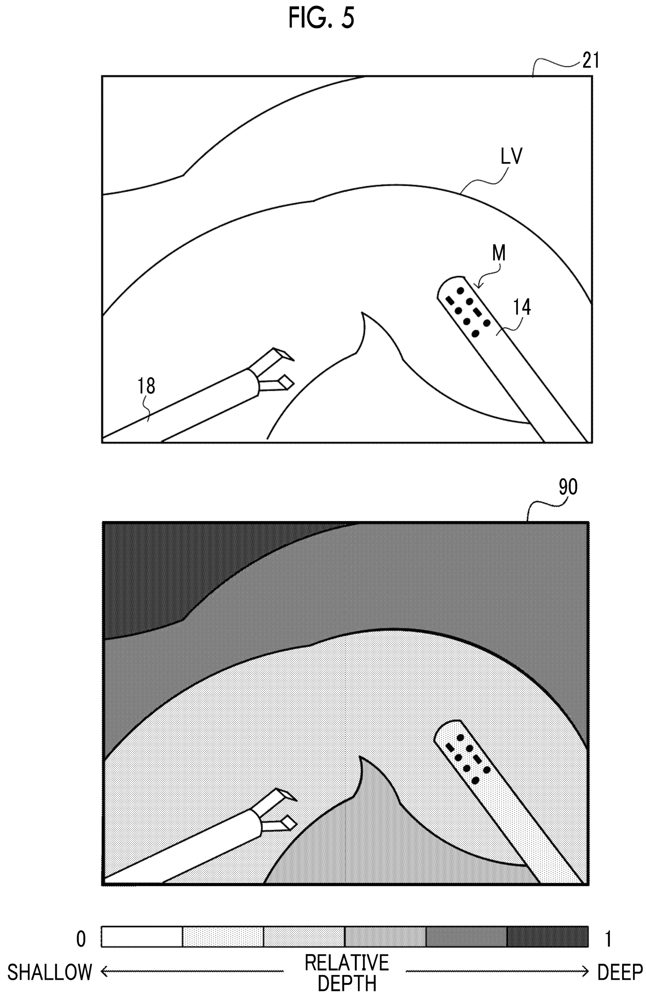

The generation unit 51 generates a relative depth map 90 in which a relative depth is assigned for each region of the surgical field image 21 based on the surgical field image 21. FIG. 5 shows an example of the surgical field image 21 and the relative depth map 90. In the example of FIG. 5, the liver LV, the ultrasound probe 14, and the treatment tool 18 (forceps) are shown in the surgical field image 21. A marker M for estimating a position and an orientation of the ultrasound probe 14 in the surgical field SF is assigned to the ultrasound probe 14.

The "region" of the surgical field image 21 means a unit obtained by dividing the surgical field image 21 based on a predetermined criterion. For example, the generation unit 51 may divide the surgical field image 21 into grids of a certain size and regard each grid as one region. As a minimum unit in this case, the region division in units of pixels is also possible. In addition, for example, the generation unit 51 may regard a group of pixels having similar relative depths as one region. In addition, for example, the generation unit 51 may regard each structure included in the surgical field image 21, such as an organ, a blood vessel, and a medical instrument, as one region. In this case, the generation unit 51 may extract the region of each structure from the surgical field image 21 by using a known segmentation technology.

The term "relative depth map" refers to a map in which a relative value corresponding to the depth is assigned to each region. The relative depth map 90 makes it possible to understand a front-and-back relationship for each region. In the relative depth map 90, for example, a value indicating a relative relationship such as "0 in front and 1 in back" is output, but the value itself does not have a physical meaning. Therefore, in the relative depth map 90, in a case where the value is changed by the movement of the medical instrument, a case may occur in which a value of a surface of an organ that is not supposed to move is also changed. In the example of FIG. 5, the relative depth map 90 is shown in which the relative depth defined in a range of 0 to 1 is visualized by a color gradation.

As a method of generating the relative depth map 90, a known technology can be applied as appropriate. For example, the estimation unit 52 may use a neural network model such as a convolutional neural network (CNN) that has been trained in advance by using the surgical field image 21 as an input and the relative depth map 90 of the input surgical field image 21 as an output.

The estimation unit 52 estimates a marker depth that is an absolute depth of the marker M shown in the surgical field image 21, based on the surgical field image 21. The marker M is a marker that can be recognized from the surgical field image 21 optically captured by the camera 13B of the endoscope 13, and is assigned to the medical instrument. In addition, information about a shape of the marker M and placement of the marker M on the medical instrument is known, and is stored in advance in the storage 43 as the dimension information 45. As the marker M, for example, a marker for estimating the position and the orientation of the ultrasound probe 14 in the surgical field SF can be used.

Specifically, the estimation unit 52 detects the marker M by searching for a morphological feature of the marker M from the surgical field image 21. For example, the estimation unit 52 may detect the marker M by using an image processing method such as pattern matching. In addition, for example, the estimation unit 52 may detect the marker M by using an artificial intelligence (AI) technology using a machine learning model. As such a machine learning model, for example, a neural network model such as a CNN that has been trained in advance by using the surgical field image 21 as an input and a region of the marker M in the input surgical field image 21 as an output can be applied.

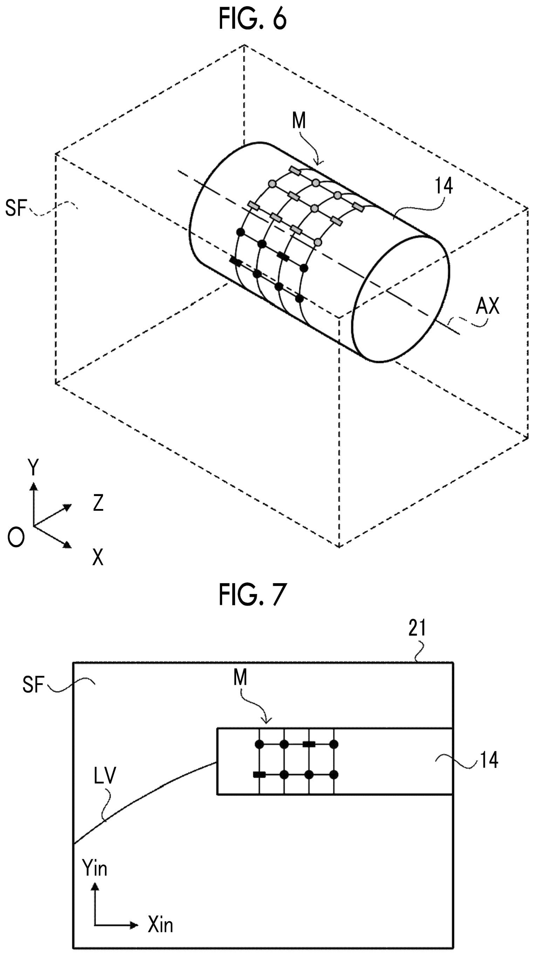

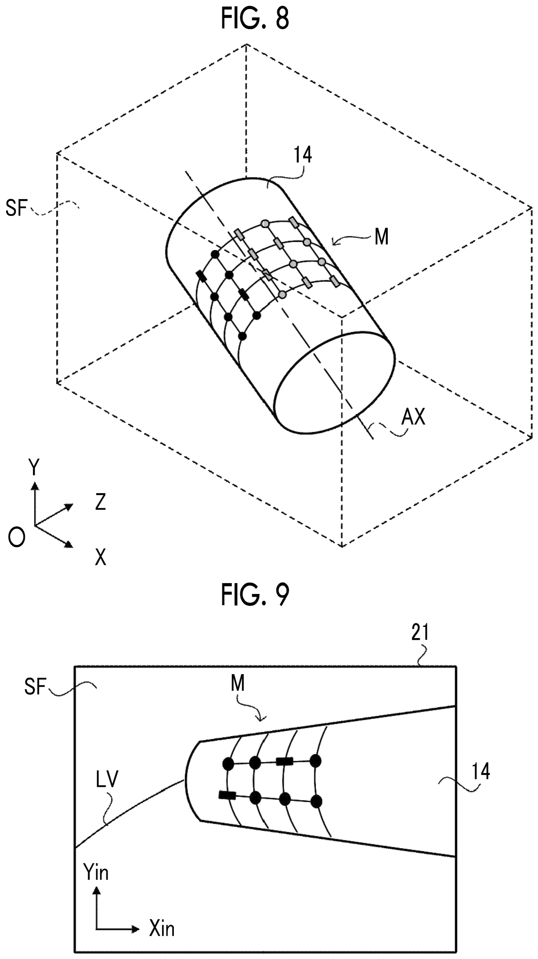

Then, the estimation unit 52 estimates the marker depth based on the detected marker M. A method of estimating the marker depth based on the marker M will be described with reference to FIGS. 6 to 9. FIGS. 6 and 8 are diagrams conceptually showing a distal end part of the ultrasound probe 14 in the surgical field SF defined as a three-dimensional space, each of which assumes a different position and orientation. FIG. 7 shows a surgical field image 21 captured in a case where the position and the orientation of the distal end part of the ultrasound probe 14 are in the state of FIG. 6. FIG. 9 shows a surgical field image 21 captured in a case where the position and the orientation of the distal end part of the ultrasound probe 14 are in the state of FIG. 8.

As an example, the marker M is a marker of a lattice pattern composed of a first line extending in a direction of an axis AX of the distal end part of the ultrasound probe 14 and a second line orthogonal to the direction of the axis AX of the distal end part and formed in the circumferential direction along an outer peripheral surface of the distal end part. A circular symbol or a rectangular symbol is assigned to each intersection in the lattice pattern. In each drawing, the marker M may be simplified or omitted.

The marker M may include at least one of a line forming the lattice pattern or a symbol disposed at each intersection of the lattice pattern. In addition, the symbol on the marker M can have any shape, and may be represented by figures such as a triangle, a polygon, a star shape, and various marks, or may be represented by a character or the like. In addition, as the marker M, a two-dimensional code and a geometric pattern (for example, ArUco and ChArUco) may be used.

In the surgical field SF of the three-dimensional space shown in FIGS. 6 and 8, a Z-axis is a direction parallel to an imaging optical axis of the camera 13B of the endoscope 13. In addition, an X-Y plane in the surgical field SF is a plane parallel to an imaging plane (Xin-Yin plane) of the camera 13B and is orthogonal to the imaging optical axis. That is, the surgical field image 21 corresponds to a projection image obtained by projecting the surgical field SF from one viewpoint O. In FIGS. 6 and 8, among the symbols constituting the marker M, a symbol reflected in the surgical field image 21 is shown in a dark color, and a symbol not reflected in the surgical field image 21 is shown in a light color.

An imaging distance from the camera 13B to the marker M (that is, a distance in a Z-axis direction parallel to the imaging optical axis) can be calculated based on a focal length of the camera 13B and the size of the marker M shown in the surgical field image 21. Specifically, by associating the size of the marker M detected from the surgical field image 21 with the information on the marker M included in the dimension information 45, the amount of change in the projection scale can be estimated, and the imaging distance can be calculated. For example, the apparent size of the marker M is smaller as the distal end part is farther from the camera 13B and is larger as the distal end part is closer to the camera 13B. The estimation unit 52 estimates the imaging distance by using such a geometric relationship. The imaging distance corresponds to the marker depth.

For example, the estimation unit 52 may estimate the imaging distance at a predetermined reference point (for example, a center) of the marker M as the marker depth. In addition, for example, the estimation unit 52 may estimate an integrated value of the imaging distances at a plurality of points (for example, the respective symbols) of the marker M as the marker depth.

In addition, the estimation unit 52 may estimate the position and the orientation of the ultrasound probe 14 based on the surgical field image 21. Specifically, the estimation unit 52 may estimate the orientation of the ultrasound probe 14 in the surgical field SF based on the form of the marker M in the surgical field image 21. The orientation of the ultrasound probe 14 is detected, for example, as a direction of the axis AX of the distal end part of the ultrasound probe 14 in the surgical field SF. The direction of the axis AX is represented by, for example, an inclined angle with respect to each of an X-axis, a Y-axis, and a Z-axis.

For example, FIG. 6 shows a state in which the axis AX of the distal end part of the ultrasound probe 14 is parallel to the X-axis in the surgical field SF of the three-dimensional space. In this case, as shown in FIG. 7, the marker M shown in the surgical field image 21 has orthogonal lines of the lattice pattern parallel to the X-axis and Y-axis. In addition, the symbols forming the lattice pattern are shown in the surgical field image 21 at equal intervals.

On the other hand, FIG. 8 shows a state in which the axis AX of the distal end part of the ultrasound probe 14 is inclined with respect to the depth direction parallel to the imaging optical axis in the surgical field SF of the three-dimensional space. In this case, as shown in FIG. 9, in the marker M shown in the surgical field image 21, the farther the marker M is from the camera 13B in the depth direction, the shorter the line extending in the circumferential direction of the lattice pattern is, and the shorter the interval between the symbols forming the lattice pattern is.

As described above, the form of the marker M shown in the surgical field image 21 changes depending on the orientation of the ultrasound probe 14. The estimation unit 52 estimates the direction of the axis AX of the distal end part of the ultrasound probe 14 as the orientation based on the form (projected lattice pattern or the like) of the marker M that changes in this way in the surgical field image 21.

In addition, in a case in which the position of the ultrasound probe 14 is changed in the surgical field SF, the position of the marker M shown in the surgical field image 21 is also changed. The estimation unit 52 may estimate the position of the ultrasound probe 14 in the surgical field SF based on the position of the marker M. The position of the ultrasound probe 14 is detected, for example, as position coordinates (X,Y,Z) of a predetermined reference point (for example, a distal end position) in the ultrasound probe 14.

The estimation unit 52 may use the estimation result of the marker depth in a case of estimating the position and the orientation of the ultrasound probe 14. On the contrary, the estimation unit 52 may use the estimation result of the position and the orientation of the ultrasound probe 14 in a case of estimating the marker depth.

The conversion unit 53 converts at least a part of the relative depths included in the relative depth map 90 into an absolute depth based on the marker depth. Examples of a method of converting the relative depth into the absolute depth include a method of multiplying a ratio (marker depth/relative depth) of the marker depth to the value of the relative depth in the region of the marker M by a scale factor.

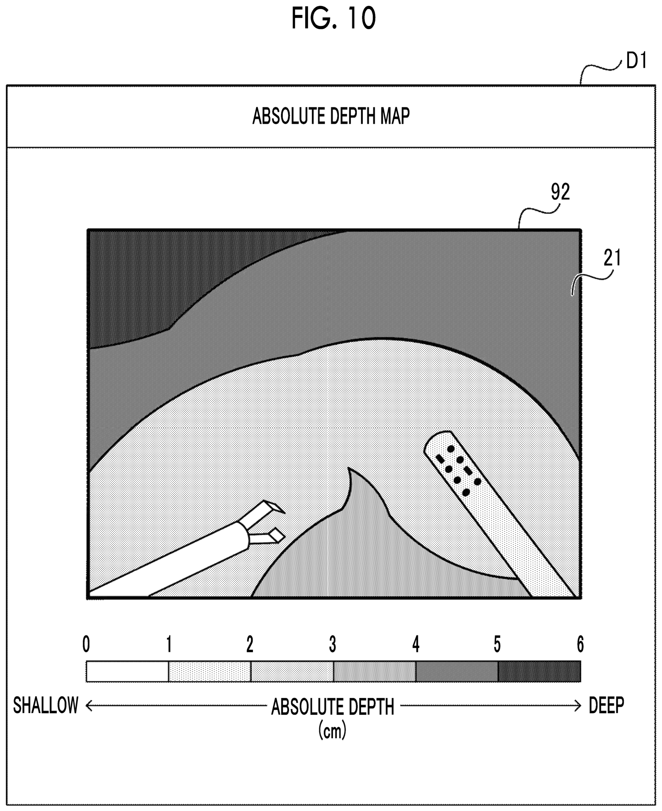

Specifically, the conversion unit 53 generates an absolute depth map 92 in which the absolute depth is assigned for each region of the surgical field image 21 by converting the relative depth of each region included in the relative depth map 90 into the absolute depth based on the relative depth map 90 and the marker depth. FIG. 10 shows an example of the absolute depth map 92 generated based on the relative depth map 90 of FIG. 5. In the example of FIG. 10, the absolute depth map 92 is shown in which the absolute depth in a range of 0 to 6 cm is visualized by a color gradation.

The term "absolute depth map" refers to a map in which absolute distance information is reflected in the relative depth map 90. Specifically, a physical distance (for example, in units of cm) from the camera 13B is assigned to each region of the absolute depth map 92. For example, it is assumed that the value of the region of the marker M in the relative depth map 90 is 1, the values of the other regions are 0.1, 0.3, and 0.6, and the marker depth is 10 cm. In this case, the absolute depths of the regions other than the marker M in the relative depth map 90 are converted into 1 cm, 3 cm, and 6 cm. On the other hand, in a case where the marker depth is 20 cm, even in a case where the values in the relative depth map 90 are the same, the absolute depths of the regions other than the marker M are converted into 2 cm, 6 cm, and 12 cm.

The surgical field SF may include a plurality of markers M. Here, the term "plurality of markers M" means the following cases. First, there is a case where a plurality of markers M (for example, a single figure) consisting of one symbol are present in the surgical field SF. Second, there is a case where only one marker M consisting of a plurality of symbols is present in the surgical field SF, and each symbol is treated as an individual marker M. Third, there is a case where a plurality of markers M consisting of a plurality of symbols are present in the surgical field SF. In the third case, the symbols in each marker M may be treated as an individual marker M.

In the first case and the third case, the plurality of markers M may be assigned to different medical instruments. For example, the ultrasound probe 14 and the treatment tool 18 may both have markers M of different forms. It is preferable that the plurality of markers M have unique forms and be identifiable.

In this case, it is preferable that the estimation unit 52 estimate the marker depth for each marker M based on the surgical field image 21. It is preferable that the conversion unit 53 convert at least a part of the relative depths included in the relative depth map 90 into the absolute depth based on the plurality of marker depths. For example, the conversion unit 53 calculates a scale factor for each marker M by associating the relative depth with the marker depth for each region of the marker M in the relative depth map 90. Then, the conversion unit 53 may obtain the absolute depth map 92 by integrating the scale factors using, for example, linear regression and a least-squares method, and applying the integrated scale factor to the entire relative depth map 90.

The conversion from the relative depth to the absolute depth can be performed by using at least one marker depth, but, by using the plurality of marker depths as described above, errors can be reduced and the conversion accuracy can be improved. For example, by referring to a plurality of markers M at different depths, it is easy to correct scale errors across the entire surgical field image 21, and thus the estimation accuracy of the depth in a wide range of the surgical field SF can be improved.

The display controller 54 causes the display 16 to display the surgical field image 21 and the absolute depth map 92 side by side or in a superimposed manner. In the example of FIG. 10, a screen D1 displayed on the display 16 shows the absolute depth map 92 superimposed and displayed on the surgical field image 21.

In addition, the display controller 54 may perform control of displaying the surgical field image 21 and the ultrasound image 22 on the display 16. In this case, the display controller 54 may perform control of displaying the surgical field image 21, the ultrasound image 22, and the absolute depth map 92 on one display 16 in a combined manner or in a switchable manner. In addition, in a case where there are a plurality of the displays 16, the display controller 54 may perform control of displaying the surgical field image 21, the ultrasound image 22, and the absolute depth map 92 on different displays 16. In addition, the display controller 54 may receive designation of a target to be displayed on the display 16 among the surgical field image 21, the ultrasound image 22, and the absolute depth map 92, and may perform control of displaying the designated target on at least one display 16.

In addition, the display controller 54 may perform control of displaying at least one of the surgical field image 21 or the ultrasound image 22 on the display 16 in a live view. The live view display refers to displaying an image generated at a predetermined frame rate based on a signal output by an imaging device for imaging a target, as a video image in real time. The imaging device includes, for example, an image sensor that optically images a target included in the endoscope 13, and the ultrasound transducer that images the target using ultrasound waves.

Next, modification examples that can be applied to the above-described embodiment will be described. It should be noted that some or all of modification examples shown below can be combined as appropriate.

### Modification Example 1

In a case where the surgical field SF includes a plurality of markers M, a part of the markers M may be unsuitable for estimating the marker depth (absolute depth). For example, in a case where the marker M is imaged near an end of the field of view of the camera 13B, or in a case where the imaging distance is long (that is, far from the camera 13B), distortion and noise may easily occur in the marker M in the surgical field image 21. In this case, it is difficult to accurately estimate the depth.

Therefore, the estimation unit 52 may estimate the marker depth for each marker M based on the surgical field image 21 and estimate validity of each marker depth. The term "validity" refers to an indicator indicating reliability of the marker depth. The validity is used to weight the marker depth, and is set such that the higher validity contributes more to the derivation of the absolute depth.

Specific methods for estimating the validity include the following criteria. First, the estimation unit 52 may estimate the validity based on a position within the screen. For example, the validity may be set to be higher as the marker M is closer to the center of the screen and lower as the marker M is closer to the end of the screen. This is because the closer to the end of the screen, the more susceptible to camera distortion, and the larger the estimation error of the marker depth.

Second, the estimation unit 52 may estimate the validity based on the depth from the camera 13B. For example, the validity may be set to be higher as the marker M is closer to the camera 13B and lower as the marker M is farther from the camera 13B. This is because the marker M is less likely to be detected from the surgical field image 21 as the depth from the camera 13B is larger (farther), and thus the estimation error of the marker depth is likely to be large.

Third, in a case where the marker M is composed of a plurality of symbols, the estimation unit 52 may estimate the validity based on the number of detectable symbols. For example, the validity may be set to be higher as the number of detectable symbols is larger.

Fourth, in a case of estimating the marker depth over a plurality of frames (see Modification Example 4 described below), the estimation unit 52 may estimate the validity based on a detection level of the marker M between the frames. For example, the validity may be set to be higher as the marker M is stably detected over a larger number of frames. In addition, for example, the validity may be set to be lower for the marker M of which the detection result is greatly different between the frames.

Fifth, in a case where different types of markers M are used in combination, the estimation unit 52 may estimate the validity based on the type of the marker M. For example, the validity may be set to be higher for the marker M having a complicated shape and/or arrangement and lower for the marker M having a simple shape and/or arrangement.

The conversion unit 53 converts at least a part of the relative depths included in the relative depth map 90 into the absolute depth based on the plurality of marker depths and the validity. For example, the conversion unit 53 calculates a scale factor for each marker M by associating the relative depth with the marker depth for each region of the marker M in the relative depth map 90. Then, the conversion unit 53 integrates the scale factors using, for example, linear regression and a least-squares method, and uses the validity as a weighting factor in the integration. Then, the conversion unit 53 obtains a highly accurate absolute depth map 92 by applying the integrated scale factor to the entire relative depth map 90.

By giving priority to the use of a marker depth with high validity, the accuracy and robustness of the depth estimation can be improved as compared to a case of equally referring to each marker depth and a case of referring to only a single marker depth. In addition, errors can be reduced in a region near the end of the field of view of the camera 13B and a region far from the camera 13B.

### Modification Example 2

In a case where the markers M are assigned to different medical instruments, a plurality of types of markers M may be shown in the surgical field image 21. In this case, the estimation accuracy of the marker depth may be improved by discriminating which medical instrument the marker M is assigned to.

Therefore, the estimation unit 52 may acquire the dimension information 45 for each medical instrument, and estimate the marker depth based on the surgical field image 21 and the dimension information 45. For example, in a case where the marker M is provided on both the ultrasound probe 14 and the treatment tool 18, the estimation unit 52 may identify the marker M by referring to a size and a shape unique to each medical instrument from the dimension information 45, and then estimate the marker depth.

As described above, by estimating the marker depth after identifying the marker M using the dimension information 45, the conversion from the relative depth to the absolute depth can be performed with high accuracy and efficiency even in the surgical field SF in which a plurality of types of medical instruments are used in combination.

### Modification Example 3

In order to improve the estimation accuracy of the marker depth, it is also effective to narrow down a region in the surgical field image 21 where the marker M may be included. Therefore, the estimation unit 52 may acquire presence information regarding a region in which each medical instrument is present in the surgical field image 21. The term "presence information" refers to information about a position, a range, and an orientation of the medical instrument in the surgical field image 21. For example, the position may include coordinates in the surgical field SF. The range may include information such as a segmentation result and a bounding box. The orientation may include a direction, a rotation angle of the medical instrument, and the like. In addition, the presence information may include information on whether or not the medical instrument is present in the surgical field image 21, information on whether or not the medical instrument is blocked by another object, and the like.

The presence information may be acquired by, for example, the estimation unit 52 analyzing the surgical field image 21 and identifying the position, the range, and the orientation of the medical instrument in the surgical field image 21. In addition, for example, the estimation unit 52 may acquire the presence information that has been generated in advance from the surgical field image 21 by an external device for generating the presence information. As a method of analyzing the presence information from the surgical field image 21, a known technology can be applied as appropriate.

The estimation unit 52 estimates the marker depth based on the surgical field image 21 and the presence information. Since the region of the medical instrument to which the marker M is assigned in the surgical field image 21 can be identified by the presence information, the erroneous recognition of the marker M can be suppressed. As a result, the conversion from the relative depth to the absolute depth can be performed with high accuracy and efficiency even in the surgical field SF in which a plurality of types of medical instruments are used in combination.

### Modification Example 4

As described above, the medical support device 11 can display the surgical field image 21 in a live view. In response to this, the processor 41 may update the absolute depth map 92 in real time or at a predetermined time interval.

Specifically, the acquisition unit 50 may acquire a plurality of surgical field images 21 that are temporally different. Hereinafter, each of the surgical field images 21 obtained by capturing the same surgical field SF in time series will be referred to as a frame. The generation unit 51 generates the relative depth map 90 for each of the surgical field images 21 (frames) that are temporally different. The estimation unit 52 estimates the marker depth for each surgical field image 21 (frame). The conversion unit 53 converts at least a part of the relative depths included in each relative depth map 90 into the absolute depth based on the marker depth estimated for each surgical field image 21 (frame).

It is assumed that the absolute depth also changes between frames for a region that is moved during the operation, such as the ultrasound probe 14, the treatment tool 18, and the target part (liver LV or the like). Therefore, it is desirable to prioritize real-time performance by updating the absolute depth for each frame. On the other hand, it is assumed that the absolute depth undergoes almost no change between frames for the trocar 17 and a region such as an organ and a blood vessel other than the target part. Therefore, it is desirable to reduce the influence of noise and estimation errors and to estimate a stable absolute depth.

Therefore, the conversion unit 53 may integrate the absolute depths in a plurality of frames for a region in which the absolute depth undergoes almost no change. Specifically, the conversion unit 53 may calculate an amount of variation in a corresponding absolute depth across the plurality of surgical field images 21 (frames) and determine whether or not the amount of variation is equal to or less than a predetermined threshold value. In a case where the amount of variation is equal to or less than the threshold value, the conversion unit 53 may derive a single absolute depth based on the absolute depth converted for each of the surgical field images 21 (frames).

In addition, the estimation unit 52 may estimate the validity of the absolute depth converted for each of the surgical field images 21 (frames). As the validity in this case, the same validity as in Modification Example 1 may be used, or the validity may be estimated based on the clarity of the surgical field image 21. For example, the validity may be set to be higher for the absolute depth converted from a frame that is clearly shown and lower for the absolute depth converted from a frame that is unclear due to shake or the like.

In a case where the amount of variation in the absolute depth is equal to or less than the predetermined threshold value, the conversion unit 53 may derive a single absolute depth based on the absolute depth converted for each of the surgical field images 21 (frames) and the validity. As described above, by integrating the absolute depths in consideration of the validity for each frame, the accuracy and robustness of the depth estimation can be improved as compared to a case of equally referring to the absolute depth of each frame and a case of referring to only the absolute depth of a single frame.

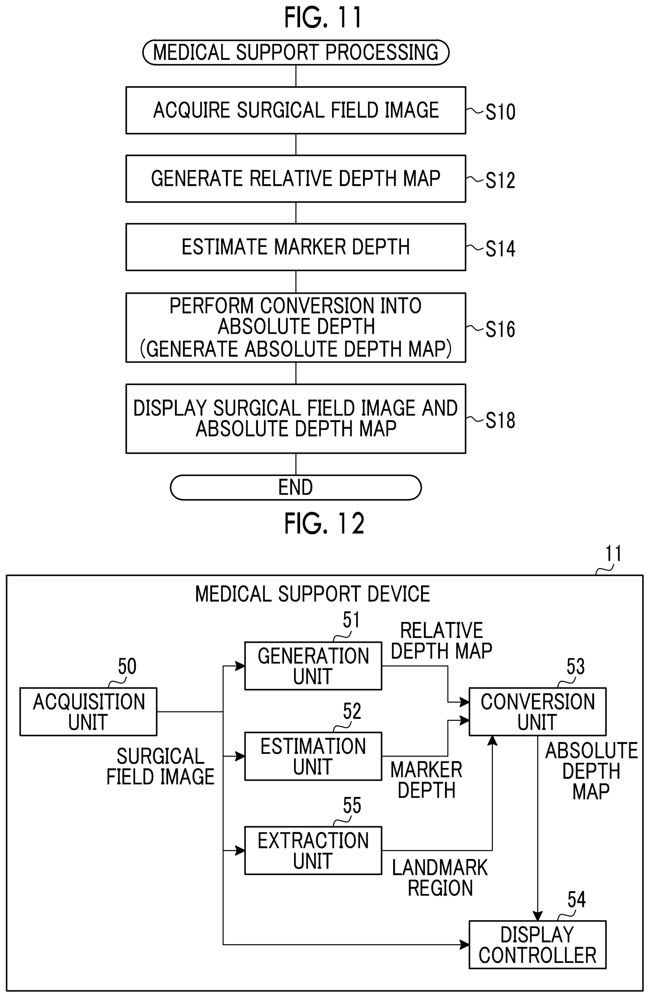

Next, an operation of the medical support device 11 according to the present embodiment will be described with reference to FIG. 11. In the medical support device 11, the medical support processing shown in FIG. 11 is executed by the processor 41 executing the medical support program 44. This processing is executed, for example, in a case where a user gives an instruction to start execution via the reception device 46.

In step S10, the acquisition unit 50 acquires the surgical field image 21 obtained by capturing the surgical field SF with the camera 13B. In step S12, the generation unit 51 generates the relative depth map 90 in which a relative depth is assigned for each region of the surgical field image 21 based on the surgical field image 21. In step S14, the estimation unit 52 estimates a marker depth that is an absolute depth of the marker M shown in the surgical field image 21, based on the surgical field image 21.

In step S16, the conversion unit 53 converts at least a part of the relative depths included in the relative depth map 90 into an absolute depth based on the marker depth. Specifically, the conversion unit 53 generates the absolute depth map 92 by converting the relative depth of each region included in the relative depth map 90 into the absolute depth based on the relative depth map 90 and the marker depth. In step S18, the display controller 54 performs control of displaying the surgical field image 21 and the absolute depth map 92 on the display 16, and ends this processing.

As described above, the medical support device 11 according to the present embodiment comprises the processor 41. The processor 41 acquires the surgical field image 21 obtained by capturing the surgical field SF with the camera 13B. In addition, the processor 41 generates the relative depth map 90 in which a relative depth is assigned for each region of the surgical field image 21 based on the surgical field image 21. In addition, the processor 41 estimates a marker depth that is an absolute depth of the marker M shown in the surgical field image 21, based on the surgical field image 21. In addition, the processor 41 converts at least a part of the relative depths included in the relative depth map 90 into an absolute depth based on the marker depth.

That is, with the medical support device 11 according to the present embodiment, the conversion from the relative depth to the absolute depth can be accurately performed even in a region such as a surface of an organ to which the marker M is not assigned, thereby supporting understanding of the spatial configuration of the entire surgical field SF. In addition, as compared with the related art method using the geometric modeling of the medical instrument, the influence of the position and the orientation of the medical instrument and/or the camera, the number of the medical instruments, and the like can be reduced, and the robustness and the general-purpose properties can be improved.

### Second Embodiment

Next, the medical support device 11 according to a second embodiment will be described. The medical support device 11 according to the present embodiment has a function of performing the conversion of the absolute depth using a landmark 80 included in the surgical field image 21, in addition to the functions of the medical support device 11 according to the first embodiment. The landmark 80 can be used instead of the marker M as a reference in a case of converting the relative depth into the absolute depth. In the following description, a part of the description overlapping with the first embodiment will be omitted.

FIG. 12 is a block diagram showing an example of a functional configuration of the medical support device 11 according to the present embodiment. The medical support device 11 comprises an acquisition unit 50, a generation unit 51, an estimation unit 52, a conversion unit 53, a display controller 54, and an extraction unit 55 as functional units. The acquisition unit 50, the generation unit 51, the estimation unit 52, the conversion unit 53, and the display controller 54 have the same functions as those in the first embodiment. The processor 41 reads out the medical support program 44 from the storage 43 and executes the medical support program 44 on the RAM 42, so that the processor 41 functions as each functional unit.

The extraction unit 55 extracts the landmark 80 from the surgical field image 21. It is assumed that both the marker M and the landmark 80 are shown in the surgical field image 21 at the time point of the extraction. In addition, the extraction unit 55 outputs information indicating a region (hereinafter, referred to as a "landmark region") corresponding to the landmark 80 in the surgical field image 21 to the conversion unit 53.

The term "landmark" refers to a structure or a feature point in the surgical field image 21 that is easy to identify and can serve as a reference point for the absolute depth. Specifically, a landmark that is difficult to move in the surgical field SF and can be recognized as the same landmark 80 even in a plurality of surgical field images 21 (for example, different viewpoints or frames) is preferable. For example, an organ such as a liver, a gallbladder, and a blood vessel, as well as a characteristic shape and a surface structure such as a mesentery, or a medical instrument such as the trocar 17 may be used as the landmark 80.

In a situation in which the marker M can be normally detected, the absolute depth in any region in the surgical field image 21 can be derived based on the relative depth map 90 and the marker depth. Therefore, the conversion unit 53 derives a landmark depth by converting the relative depth of the landmark region included in the relative depth map 90 into the absolute depth based on the marker depth. In addition, the conversion unit 53 stores the landmark depth in association with information (for example, a shape, a size, and a position) on the landmark 80 in the storage 43 or the like.

Next, the conversion unit 53 converts the relative depth of at least a part of regions other than the landmark region included in the relative depth map 90 into the absolute depth based on the landmark depth. For example, the conversion unit 53 converts the relative depth in any region into the absolute depth by multiplying a ratio (landmark depth/relative depth) of the landmark depth to the value of the relative depth in the landmark region by a scale factor.

Specifically, in a case where the marker depth is not able to be estimated, the conversion unit 53 may convert the relative depth of at least a part of regions other than the landmark region included in the relative depth map 90 into the absolute depth based on the landmark depth. The "case where the marker depth is not able to be estimated" includes, for example, a case where the marker M and/or the camera 13B is moved or the marker M is blocked by an organ or the like so that the marker M is not shown in the surgical field image 21. In addition, for example, it includes a case where the marker M is blurred or an illumination condition is poor (reflection, shadow, insufficient illuminance, or the like), and thus the marker M cannot be detected from the surgical field image 21.

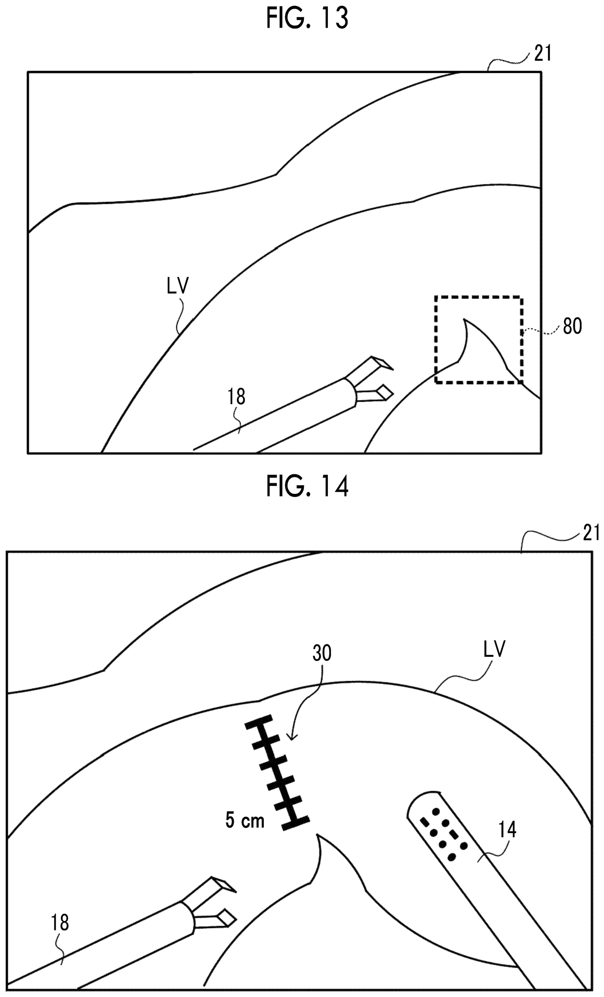

As shown in FIG. 13, in a case where the viewpoint of the camera 13B is moved, the conversion unit 53 may correct the landmark depth. FIG. 13 shows a state in which the marker M cannot be detected due to the movement of the viewpoint of the camera 13B that captures the surgical field image 21 from the viewpoint of FIG. 5. In this case, the conversion unit 53 corrects the landmark depth (absolute depth) by comparing the apparent shape, size, position, and the like of the landmark 80 in the surgical field image 21 between the frame at the time point of deriving the landmark depth and the current frame. Then, the conversion unit 53 converts the relative depth into the absolute depth by using a scale factor using the corrected landmark depth.

In a case where the viewpoint of the camera 13B is not moved, the positional relationship between the camera 13B and the landmark 80 does not change, so that the landmark depth also does not change. Therefore, the conversion unit 53 may convert the relative depth into the absolute depth by using a scale factor using the landmark depth derived in the past frame as it is.

In addition, the conversion unit 53 may convert the relative depth into the absolute depth based on the landmark depth even in a case where the validity of the estimated marker depth is lower than a predetermined threshold value.

As described above, the processor 41 of the medical support device 11 according to the present embodiment extracts the landmark 80 from the surgical field image 21. In addition, the processor 41 derives the landmark depth by converting the relative depth of the region that is included in the relative depth map 90 and that corresponds to the landmark 80 into the absolute depth based on the marker depth. In addition, the processor 41 converts the relative depth of at least a part of regions other than the region that is included in the relative depth map 90 and that corresponds to the landmark 80 into the absolute depth based on the landmark depth.

That is, with the medical support device 11 according to the present embodiment, even in a case where the marker M is not shown in the surgical field image 21, the relative depth can be converted into the absolute depth using the landmark 80. As a result, in comparison with the first embodiment, the depth estimation can be performed under a wider range of conditions, enabling more stable support for understanding of the spatial configuration of the entire surgical field SF.

### Third Embodiment

Next, the medical support device 11 according to a third embodiment will be described. The medical support device 11 according to the present embodiment provides support information corresponding to scale information of the space (surgical field SF) shown in the surgical field image 21, in addition to the functions of the medical support device 11 according to the first embodiment and/or the second embodiment. In the following description, a part of the description overlapping with the first embodiment or the second embodiment will be omitted.

The term "scale information" refers to information for defining a correspondence relationship between three-dimensional information (for example, XYZ coordinates) estimated from the surgical field image 21 and a distance in an actual physical space (surgical field SF). The scale information is defined for each of the X direction, the Y direction, and the Z direction.

The scale information in the X direction and the Y direction is defined, for example, by a length (cm/pixel) per pixel in the surgical field image 21. Specifically, the estimation unit 52 may detect two markers M whose actual distance is known from the surgical field image 21 and derive the scale information in the X direction and the Y direction by associating the pixel distance on the surgical field image 21 with the actual distance.

The scale information in the Z direction can be estimated based on, for example, a difference between the absolute depths (marker depths) of at least two markers M. Specifically, the estimation unit 52 may estimate at least two marker depths based on the surgical field image 21 and derive the scale information based on the at least two marker depths. For example, the estimation unit 52 may derive the scale information in the Z direction by associating the difference in the marker depth with the actual distance for two markers M whose actual distance is known.

In a case of deriving each scale information, the estimation unit 52 may correct optical distortion of the camera 13B. For example, the estimation unit 52 may derive different scale information near the center and at an end part of the surgical field image 21 by taking into consideration lens distortion correction parameters.

The display controller 54 performs control of displaying information corresponding to the scale information. The "information corresponding to the scale information" is, for example, gradations and guidelines. As an example, the display controller 54 may extract a superimposition object from the surgical field image 21 and performs control of superimposing and displaying gradations 30 related to the superimposition object on the surgical field image 21 based on the absolute depth and the scale information. As a method of extracting the superimposition object, a known technology can be applied as appropriate.

The term "superimposition object" means a structure or a region that is a target for superimposing the information such as the gradations 30. Examples of the superimposition object include structures such as various medical instruments, organs, tissues, and tumors, and a gap between the structures. That is, the display controller 54 may visualize a size of the structure by displaying the gradations 30 along the various structures, or may visualize a distance by displaying the gradations 30 in the gap between the structures.

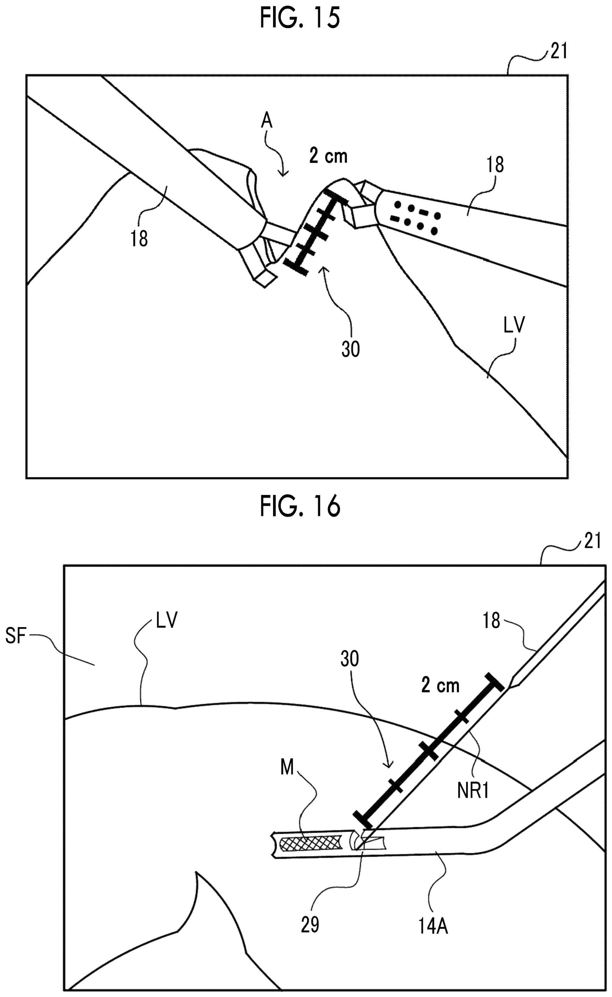

As an example, FIG. 14 shows an example of superimposing and displaying the gradations 30 indicating a size of the liver LV on the liver LV in the surgical field image 21. FIG. 15 shows an example of superimposing and displaying the gradations 30 indicating a size of a resection site A on the resection site A in the surgical field image 21.

FIG. 16 shows an example of superimposing and displaying the gradations 30 indicating a distance between a distal end of the treatment tool 18 (biopsy needle) and a target on the gap in the surgical field image 21. FIG. 16 shows a state in which the biopsy needle is inserted into a guide groove 29. The guide groove 29 is a groove for guiding the insertion of the biopsy needle into a target position (for example, a tumor) inside an organ, and is provided in the insertion part 14A of the ultrasound probe 14. In this case, the display controller 54 may identify a puncture path NR1 based on the absolute depth of the biopsy needle and superimpose and display the puncture path NR1 on the surgical field image 21.

In addition, for example, the gradations 30 indicating a distance between the medical instrument and a site (for example, a blood vessel, a nerve, and a cavity wall) to be avoided from contact may be used. In addition, for example, the display controller 54 may display not only gradations 30 relating to a specific structure, but also gradations 30 for visualizing the relative distance of the surgical field image 21 at, for example, the four corners or center of the surgical field image 21.

As described above, the processor 41 of the medical support device 11 according to the present embodiment estimates at least two marker depths based on the surgical field image 21 and derives the scale information of the space (surgical field SF) shown in the surgical field image 21 based on the at least two marker depths. In addition, the processor 41 performs control of displaying the information corresponding to the scale information.

That is, with the medical support device 11 according to the present embodiment, the sense of scale in the plane direction and the depth direction of the surgical field SF can be visually provided, so that understanding of the spatial configuration of the entire surgical field SF can be effectively supported.

### Fourth Embodiment

Next, the medical support device 11 according to a fourth embodiment will be described. The medical support device 11 according to the present embodiment provides support information using a three-dimensional image, in addition to the functions of the medical support device 11 according to the first embodiment, the second embodiment, and/or the third embodiment. In the following description, a part of the description overlapping with the first embodiment, the second embodiment, or the third embodiment will be omitted.

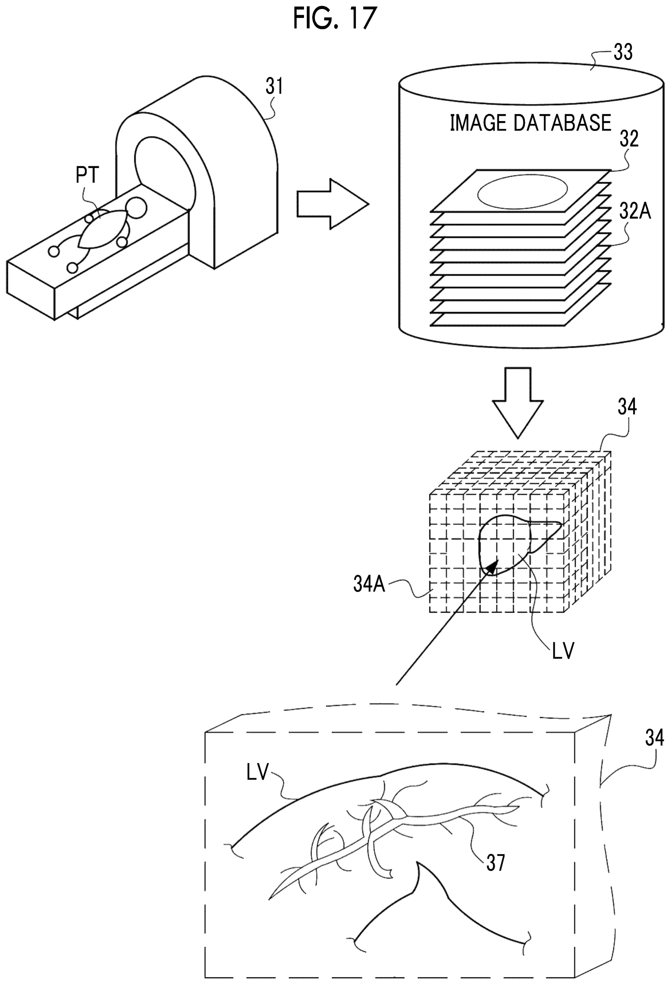

The acquisition unit 50 acquires a three-dimensional image 34 of the surgical field SF. In the present disclosure, the three-dimensional image 34 is a preoperative image. FIG. 17 shows an example of the three-dimensional image 34. The term "three-dimensional image" refers to three-dimensional data (three-dimensional model) generated based on a tomographic image group 32 captured in advance by a tomography apparatus 31 such as a computed tomography (CT) apparatus and a magnetic resonance imaging (MRI) apparatus.

For example, in a case where a CT apparatus is used as the tomography apparatus 31, a CT value is acquired while a radiation source and a radiation detector are rotated around a body axis of the patient PT. The acquisition of the CT value is performed at each position in the body axis direction by performing scanning using the radiation source and the radiation detector in the body axis direction of the patient PT. The CT value is a radiation absorption value inside the body of the patient PT.

The tomography apparatus 31 generates a tomographic image 32A by performing image reconstruction processing based on the CT value acquired in each direction around the body axis. Each tomographic image 32A is a two-dimensional image generated in accordance with a slice thickness in the body axis direction, and the tomographic image group 32 is a set of a plurality of tomographic images 32A corresponding to the respective positions in the body axis direction. The tomographic image group 32 is stored in an image database 33 such as a picture archiving and communication system (PACS). The acquisition unit 50 acquires the three-dimensional image 34 from the image database 33.

Based on the tomographic image group 32, 3D modeling is performed to numerically describe a three-dimensional shape of the patient PT, thereby generating a three-dimensional image 34 that is a set of voxel data 34A. The voxel data 34A is a unit of a pixel in the three-dimensional space, and has three-dimensional coordinate information and a pixel value.

The three-dimensional image 34 is capable of reproducing an external shape of the body of the patient PT, an anatomical site such as an organ inside the body, and an internal structure thereof. For example, FIG. 17 conceptually shows an example of three-dimensional data including the liver LV and a blood vessel structure 37.

The display controller 54 performs control of displaying various types of support information on the display 16 by using the acquired three-dimensional image 34. Hereinafter, an example of the support information will be described with reference to Examples 1 to 3. Some or all of examples shown below can be combined as appropriate.

### Example 1

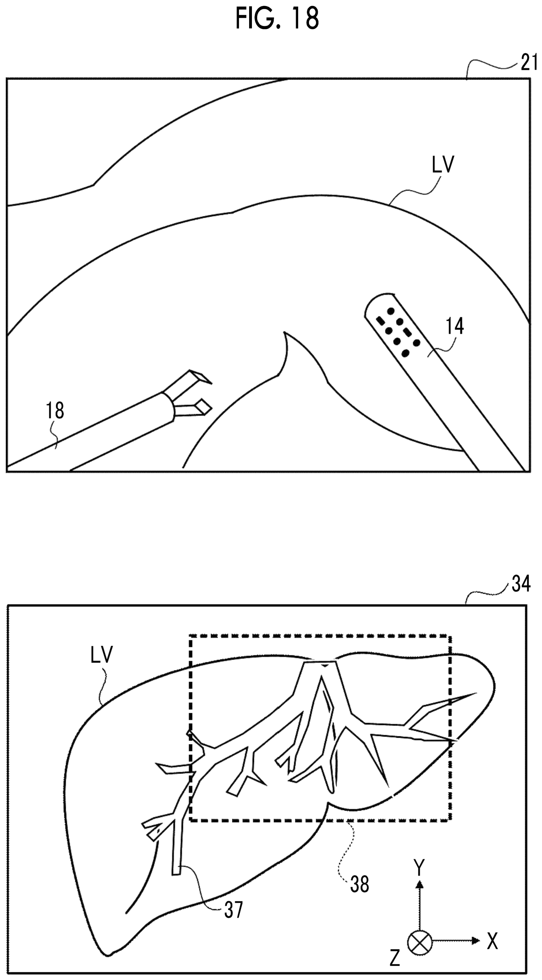

The present example will be described with reference to FIGS. 18 to 20. In the present example, a region corresponding to the field of view (FOV) of the surgical field image 21 is identified on the three-dimensional image 34 side, and the two are associated with each other, thereby effectively associating the three-dimensional image 34 obtained before surgery (preoperatively) with the surgical field image 21 captured during surgery.

The display controller 54 extracts a region of interest from the surgical field image 21. The term "region of interest" refers to a region for specifying the field of view of the surgical field image 21 in the three-dimensional image 34, and is a region that can be extracted from both the surgical field image 21 and the three-dimensional image 34. As the region of interest, for example, a region of an organ, a blood vessel, a tumor, or the like can be applied as appropriate.

As a method of extracting the region of interest, a known technology can be applied as appropriate. For example, the display controller 54 may use a neural network model such as a CNN that has been trained in advance to receive the surgical field image 21 as an input and output the region of interest from the input surgical field image 21.

In addition, the display controller 54 identifies a corresponding region that corresponds to the field of view of the surgical field image 21 in the three-dimensional image 34 based on the absolute depth and the region of interest. For example, the display controller 54 may identify the corresponding region by referring to the absolute depth of the region of interest and associating the absolute depth with each voxel in the three-dimensional image 34.

Then, the display controller 54 performs control of displaying the three-dimensional image 34 based on the corresponding region. For example, as shown in FIG. 18, the display controller 54 may emphasize the corresponding region in the three-dimensional image 34 by surrounding the corresponding region with a rectangle 38. The method of the emphasis display is not limited to this, and, for example, a color of the corresponding region may be changed, or only the corresponding region may be displayed.

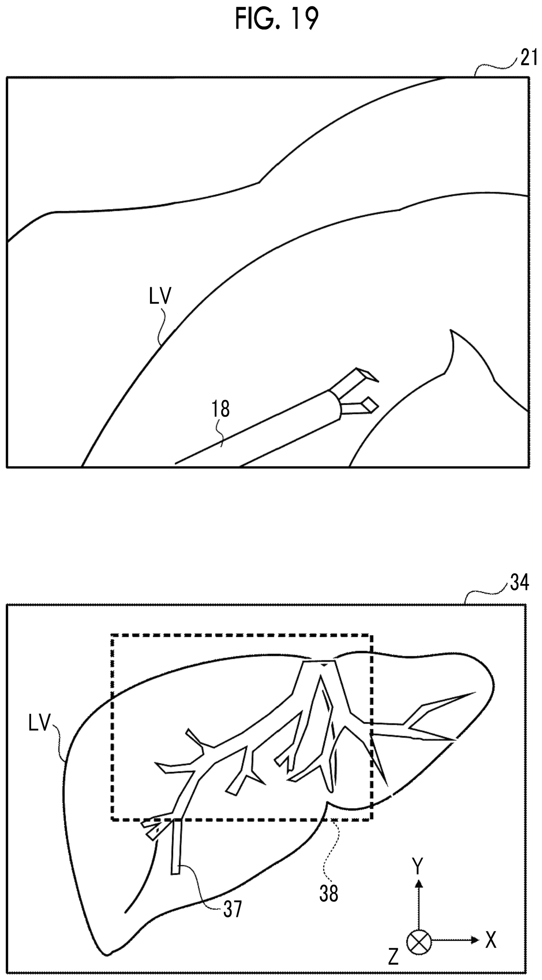

In addition, in FIG. 19, the field of view of the surgical field image 21 is different from the example of FIG. 18, and the position of the rectangle 38 on the three-dimensional image 34 is also different correspondingly. As described above, the display controller 54 may re-identify the corresponding region in the three-dimensional image 34 in accordance with the change in the field of view of the surgical field image 21 and change the region to be emphasized in the three-dimensional image 34.

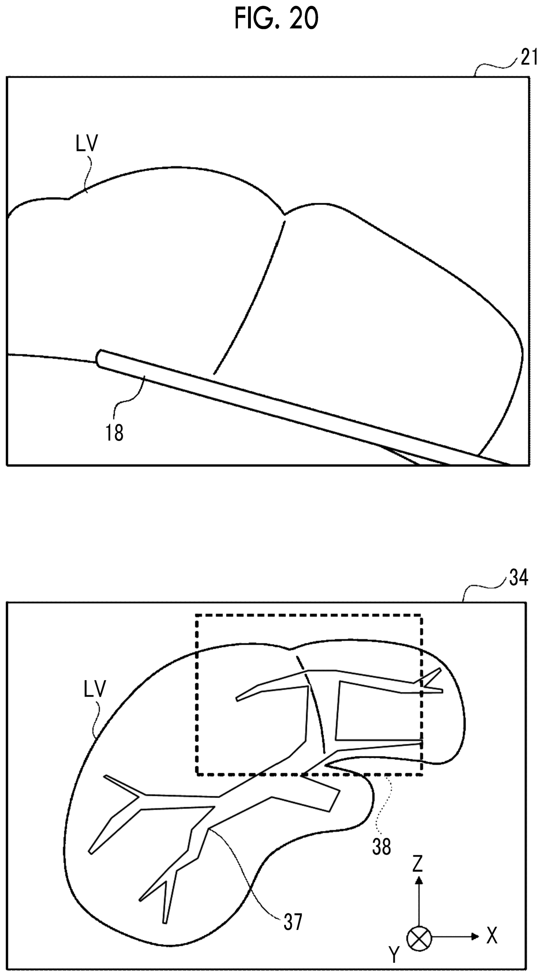

In addition, for example, the display controller 54 may display the three-dimensional image 34 at the same angle as the viewpoint of the surgical field image 21. In the example of FIG. 20, the three-dimensional image 34 is displayed at the same angle as the viewpoint of the surgical field image 21 in which the liver LV is viewed from a caudal direction. As a result, it is easy to take visual correspondence.

The display controller 54 may cause the display 16 to display the surgical field image 21 and the three-dimensional image 34 associated with each other in this way side by side.

### Example 2

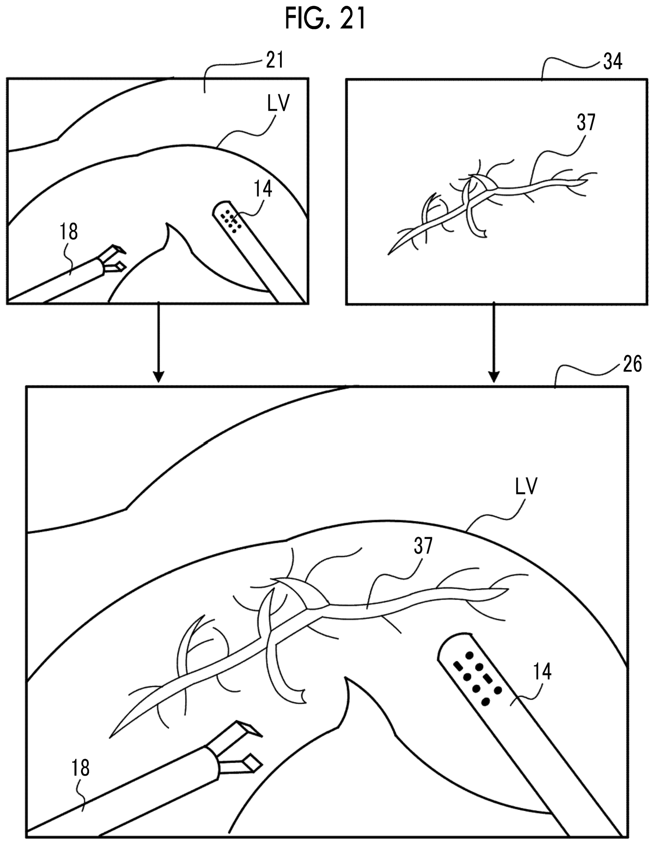

The present example will be described with reference to FIG. 21. In the present example, an internal structure that is difficult to understand with only the surgical field image 21 is visualized by superimposing and displaying the three-dimensional image 34 on the surgical field image 21.

The display controller 54 extracts a region of interest from the surgical field image 21 in the same manner as in Example 1. In addition, the display controller 54 registers the region of interest with the three-dimensional image 34 based on the absolute depth and the region of interest. For example, the display controller 54 identifies a three-dimensional shape of a surface of the region of interest based on the absolute depth, and searches for the three-dimensional shape from the three-dimensional image 34 to register the region of interest with the three-dimensional image 34. As a result, each voxel in the three-dimensional image 34 is associated with each pixel of the surgical field image 21.

Then, the display controller 54 performs control of superimposing and displaying the three-dimensional image 34 on the surgical field image 21 based on the result of the registration. In the example of FIG. 21, a superimposed image 26 in which the blood vessel structure 37 is superimposed and displayed on the surface of the liver LV in the surgical field image 21 is shown. The display controller 54 may cause the display 16 to display the superimposed image 26 in this way.

The display controller 54 may identify the corresponding region that corresponds to the field of view of the surgical field image 21 in the three-dimensional image 34 or a non-rigid deformation parameter based on the result of the registration, and perform control of superimposing and displaying the three-dimensional image 34 on the surgical field image 21 based on the result of the identification. That is, the display controller 54 may identify the corresponding region in the three-dimensional image 34 and perform control of superimposing and displaying the identified portion on the surgical field image 21.

In addition, the display controller 54 may identify a non-rigid deformation parameter in the three-dimensional image 34 based on the result of the registration, and perform control of superimposing and displaying the three-dimensional image 34 on the surgical field image 21 based on the result of the identification. The term "non-rigid deformation parameter" refers to a parameter for appropriately shape-correcting the three-dimensional image 34 to match the surgical field image 21. For example, during surgery, it is assumed that the shape of the liver LV is deformed since the three-dimensional image 34 is captured. By applying the non-rigid deformation parameter, the shape of the three-dimensional image 34 can be corrected to match such deformation of the liver LV.

### Example 3

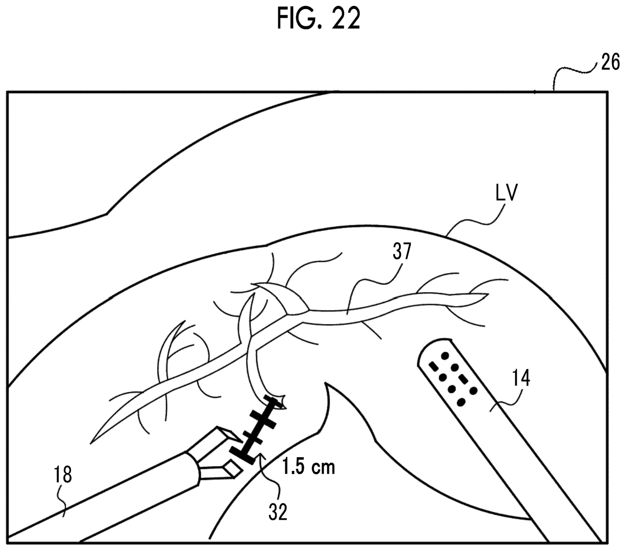

The present example will be described with reference to FIG. 22. In the present example, a proximity situation is visualized by estimating a distance between the blood vessel structure 37 included in the three-dimensional image 34 and the viewpoint of the surgical field image 21 or the treatment tool 18 or the like.

The display controller 54 identifies an adjacent blood vessel closest to the viewpoint of the surgical field image 21 based on the surgical field image 21 and the three-dimensional image 34. Specifically, the display controller 54 identifies the corresponding region corresponding to the field of view of the surgical field image 21 in the three-dimensional image 34 (see Example 1) or registers the surgical field image 21 with the three-dimensional image 34 (see Example 2). As a result, the blood vessel included in the field of view of the surgical field image 21 is limited, making it possible to identify the adjacent blood vessel closest to the viewpoint.

Then, the display controller 54 derives a distance between the adjacent blood vessel and a predetermined position based on the surgical field image 21 and the absolute depth. The term "predetermined position" refers to a position that is a starting point from which it is desired to derive the distance to the adjacent blood vessel, and is, for example, the viewpoint (camera 13B) of the surgical field image 21 and various medical instruments. The predetermined position may be different for each frame or may be movable. In the example of FIG. 22, the distance between the treatment tool 18 (forceps) and the adjacent blood vessel is displayed as gradations 32.

Specifically, the display controller 54 identifies a physical distance from the viewpoint of the surgical field image 21 to the organ surface based on the surgical field image 21 and the absolute depth. In addition, the display controller 54 identifies a physical distance from the organ surface to the adjacent blood vessel based on the three-dimensional image 34. The display controller 54 adds these together to derive a physical distance from the viewpoint of the surgical field image 21 to the adjacent blood vessel.

In a case where the physical distance from the viewpoint of the surgical field image 21 to the adjacent blood vessel is derived, a physical distance from any position (that is, a position at which the absolute depth can be identified) shown in the surgical field image 21 to the adjacent blood vessel can also be derived. Specifically, the display controller 54 need only divide the absolute depth at the position that is the starting point from which it is desired to derive the distance to the adjacent blood vessel, from the physical distance from the viewpoint of the surgical field image 21 to the adjacent blood vessel.

A form may be adopted in which the distance to the predetermined position is derived for any target part inside an organ in addition to or instead of the adjacent blood vessel. Specifically, the display controller 54 may identify the target part inside the organ based on the three-dimensional image 34. The term "target part" refers to, for example, a surgical target part such as a tumor and a site (for example, a blood vessel, a nerve, and a cavity wall) to be avoided from contact with the medical instrument. Then, the display controller 54 may derive a distance between the target part and a predetermined position based on the three-dimensional image 34, the surgical field image 21, and the absolute depth.

For example, by identifying a tumor present in the liver LV before surgery and registering the surgical field image 21 with the three-dimensional image 34 during surgery, the distance and direction to the tumor can be intuitively visualized. This can support in the operation of the treatment tool 18 and the design of the surgical plan. In addition, in a case of deriving the distance to the site to be avoided from contact, the safety can be improved.

As described above, with the medical support device 11 according to the present embodiment, by using the three-dimensional image 34, the structure of the entire surgical field SF and the structure inside the organ, which are difficult to understand with the surgical field image 21 alone, can be understood, so that understanding of the spatial configuration can be effectively supported.

In each of the above-described embodiments, a form has been described in which the marker M for estimating the position and the orientation of the ultrasound probe 14 is used to estimate the marker depth, but the present disclosure is not limited to this. As the marker M used in a case of estimating the marker depth, any marker other than the marker for estimating the position and the orientation can be applied.

In each of the above-described embodiments, a form has been described in which the position and the orientation of the ultrasound probe 14 in the surgical field SF are estimated using the marker M, but the present disclosure is not limited to this. For example, the position and the orientation may be estimated by detecting a characteristic shape of the ultrasound probe 14 from the surgical field image 21 through image analysis.

In each of the above-described embodiments, a form has been described in which the ultrasound probe 14 is used as an example of the medical instrument that is inserted into the body of the patient PT and images the internal structure of the target part, but the present disclosure is not limited to this. For example, a medical probe such as an optical coherence tomography (OCT) probe may be applied as such a medical instrument.

In each of the above-described embodiments, the body cavity such as the abdomen and the chest cavity has been described as an example of the surgical field SF, but the present disclosure is not limited to this. For example, as the surgical field SF, an upper gastrointestinal tract such as the esophagus, a lower gastrointestinal tract such as the intestine, or a tubular organ such as a bronchus may be used. In a case where the technology of the present disclosure is applied to the surgical field SF in the tubular organ, for example, the marker M may be provided on a base end part of a soft endoscope to be inserted into the tubular organ.

In each of the above-described embodiments, a form has been described in which the marker M assigned to the ultrasound probe 14 and/or the treatment tool 18 to be inserted into the body cavity is imaged by the camera 13B of the endoscope 13, but the present disclosure is not limited to this. For example, a form may be adopted in which the marker M assigned to an ultrasound probe and/or various medical tools (for example, forceps, tweezers, a scalpel, a blade, a hook, and a needle holder) that scan a body surface is imaged by a camera. For example, by using a webcam, the technology of the present disclosure can also be applied to remote surgery via the body surface.

In each of the above-described embodiments, each process is executed by any computer. In addition, any computer may execute these processes using a processor as hardware, a program as software, or a combination thereof. In that case, the processor is configured to execute various processes in the present embodiment in cooperation with the program and can function as each unit or each means in the present embodiment. Further, the execution order of the process by the processor is not limited to the order described above and may be changed as appropriate. Any computer may be a general-purpose computer, a computer for a specific use, a workstation, or another system capable of executing each process.

The processor may be configured by one or more pieces of hardware, and the type of hardware is not limited. For example, the processor can be configured with a central processing unit (CPU), a micro processing unit (MPU), a programmable logic device such as a field programmable gate array (FPGA), a dedicated circuit for executing a specific process such as an application specific integrated circuit (ASIC), or hardware such as a graphic processing unit (GPU) or a neural processing unit (NPU). In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each process executed by the processor is not limited to the above order and may be changed as appropriate. The hardware is configured by an electric circuit (circuitry) in which circuit elements such as semiconductor elements are combined.

Further, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

In addition, in each of the above-described embodiments, the medical support program 44 has been described as being stored (installed) in the storage 43 in advance, but the present disclosure is not limited to this. The medical support program 44 may be provided in a form of being recorded on a recording medium, such as a compact disc read only memory (CD-ROM), a digital versatile disc read only memory (DVD-ROM), or a universal serial bus (USB) memory. In addition, the medical support program 44 may be downloaded from an external device via a network.

The technology of the present disclosure extends to any program products. The program product includes a product in any aspect for providing a program. For example, the program product includes a program provided through a network such as the Internet, and a non-transitory computer-readable recording media such as a CD-ROM, a DVD-ROM, and a USB memory in which the program is stored.

In the technology of the present disclosure, the embodiment and the modification examples described above can be combined as appropriate. The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts according to the embodiments of the technology of the present disclosure. As a result, it goes without saying that unnecessary parts may be deleted, new elements may be added, or replacements may be made with respect to the above-described contents and the above-shown contents within a range that does not deviate from the gist of the technology of the present disclosure.

The following appendices are disclosed with regard to the above embodiment.

### Supplementary Note 1

A medical support device comprising:
a processor,
in which the processor is configured to
   acquire a surgical field image obtained by capturing a surgical field with a camera,
   generate a relative depth map in which a relative depth is assigned for each region of the surgical field image, based on the surgical field image,
   estimate a marker depth that is an absolute depth of a marker shown in the surgical field image, based on the surgical field image, and
   convert at least a part of the relative depths included in the relative depth map into an absolute depth based on the marker depth.

### Supplementary Note 2

The medical support device according to Supplementary Note 1,
in which the processor is configured to generate an absolute depth map in which an absolute depth is assigned for each region of the surgical field image, based on the relative depth map and the marker depth.

### Supplementary Note 3

The medical support device according to Supplementary Note 2,
in which the processor is configured to perform control of displaying the surgical field image and the absolute depth map side by side or in a superimposed manner.

### Supplementary Note 4

The medical support device according to any one of Supplementary Notes 1 to 3,
in which the surgical field includes a plurality of the markers, and
the processor is configured to
   estimate the marker depth for each marker based on the surgical field image, and
   convert at least a part of the relative depths included in the relative depth map into an absolute depth based on a plurality of the marker depths.

### Supplementary Note 5

The medical support device according to Supplementary Note 4,
in which the processor is configured to
estimate validity of each marker depth, and
convert at least a part of the relative depths included in the relative depth map into an absolute depth based on a plurality of the marker depths and the validity.

### Supplementary Note 6

The medical support device according to any one of Supplementary Notes 1 to 5,
in which the marker is assigned to a medical instrument, and
the processor is configured to
   acquire dimension information for each medical instrument, and
   estimate the marker depth based on the surgical field image and the dimension information.

### Supplementary Note 7

The medical support device according to any one of Supplementary Notes 1 to 6,
in which the marker is assigned to a medical instrument, and
the processor is configured to
   acquire presence information regarding a region in which each medical instrument is present in the surgical field image, and
   estimate the marker depth based on the surgical field image and the presence information.

### Supplementary Note 8

The medical support device according to any one o Supplementary Notes 1 to 7,
in which the processor is configured to
acquire a plurality of the surgical field images that are temporally different,
for each of the surgical field images, generate the relative depth map, estimate the marker depth, and perform conversion into the absolute depth,
calculate an amount of variation in a corresponding absolute depth across the plurality of surgical field images, and
derive a single absolute depth based on the absolute depth converted for each of the surgical field images in a case where the amount of variation is equal to or less than a predetermined threshold value.

### Supplementary Note 9

The medical support device according to Supplementary Note 8,
in which the processor is configured to
estimate validity of the absolute depth converted for each of the surgical field images, and
derive the single absolute depth based on the absolute depth converted for each of the surgical field images and the validity in a case where the amount of variation is equal to or less than the predetermined threshold value.

### Supplementary Note 10

The medical support device according to any one of Supplementary Notes 1 to 9,
in which the processor is configured to
extract a landmark from the surgical field image,
derive a landmark depth by converting a relative depth of a region that is included in the relative depth map and that corresponds to the landmark into an absolute depth based on the marker depth, and
convert a relative depth of at least a part of regions other than the region that is included in the relative depth map and that corresponds to the landmark into an absolute depth based on the landmark depth.

### Supplementary Note 11

The medical support device according to Supplementary Note 10,
in which the processor is configured to convert a relative depth of at least a part of regions other than the region that is included in the relative depth map and that corresponds to the landmark into an absolute depth based on the landmark depth in a case where the marker depth is not able to be estimated.

### Supplementary Note 12

The medical support device according to any one of Supplementary Notes 1 to 11,
in which the processor is configured to
estimate at least two marker depths based on the surgical field image,
derive scale information of a space shown in the surgical field image based on the at least two marker depths, and
perform control of displaying information corresponding to the scale information.

### Supplementary Note 13

The medical support device according to Supplementary Note 12,
in which the processor is configured to
extract a superimposition object from the surgical field image, and
perform control of superimposing and displaying gradations related to the superimposition object on the surgical field image based on the absolute depth and the scale information.

### Supplementary Note 14

The medical support device according to any one of Supplementary Notes 1 to 13,
in which the processor is configured to
acquire a preoperative three-dimensional image of the surgical field,
extract a region of interest from the surgical field image,
identify a corresponding region that corresponds to a field of view of the surgical field image in the preoperative three-dimensional image based on the absolute depth and the region of interest, and
perform control of displaying the preoperative three-dimensional image based on the corresponding region.

### Supplementary Note 15

The medical support device according to any one of Supplementary Notes 1 to 14,
in which the processor is configured to
acquire a preoperative three-dimensional image of the surgical field,
extract a region of interest from the surgical field image,
register the region of interest with the preoperative three-dimensional image based on the absolute depth and the region of interest, and
perform control of superimposing and displaying the preoperative three-dimensional image on the surgical field image based on a result of the registration.

### Supplementary Note 16

The medical support device according to Supplementary Note 15,
in which the processor is configured to
identify at least one of a corresponding region that corresponds to a field of view of the surgical field image in the preoperative three-dimensional image or a non-rigid deformation parameter based on the result of the registration, and
perform control of superimposing and displaying the preoperative three-dimensional image on the surgical field image based on a result of the identification.

### Supplementary Note 17

The medical support device according to any one of Supplementary Notes 1 to 16,
in which the processor is configured to
acquire a preoperative three-dimensional image of the surgical field,
identify an adjacent blood vessel closest to a viewpoint of the surgical field image based on the surgical field image and the preoperative three-dimensional image, and
derive a distance between the adjacent blood vessel and a predetermined position based on the surgical field image and the absolute depth.

### Supplementary Note 18

The medical support device according to any one of Supplementary Notes 1 to 17,
in which the processor is configured to
acquire a preoperative three-dimensional image of the surgical field,
identify a target part inside an organ based on the preoperative three-dimensional image, and
derive a distance between the target part and a predetermined position based on the preoperative three-dimensional image, the surgical field image, and the absolute depth.

### Supplementary Note 19

A medical support method executed by a computer, the medical support method comprising:
acquiring a surgical field image obtained by capturing a surgical field with a camera;
generating a relative depth map in which a relative depth is assigned for each region of the surgical field image, based on the surgical field image;
estimating a marker depth that is an absolute depth of a marker shown in the surgical field image, based on the surgical field image; and
converting at least a part of the relative depths included in the relative depth map into an absolute depth based on the marker depth.

### Supplementary Note 20

A medical support program causing a computer to execute:
acquiring a surgical field image obtained by capturing a surgical field with a camera;
generating a relative depth map in which a relative depth is assigned for each region of the surgical field image, based on the surgical field image;
estimating a marker depth that is an absolute depth of a marker shown in the surgical field image, based on the surgical field image; and
converting at least a part of the relative depths included in the relative depth map into an absolute depth based on the marker depth.

## Claims

1. A medical support device comprising a processor, wherein the processor is configured to:
acquire a surgical field image obtained by capturing a surgical field with a camera;
generate a relative depth map in which a relative depth is assigned for each region of the surgical field image, based on the surgical field image;
estimate a marker depth that is an absolute depth of a marker shown in the surgical field image, based on the surgical field image; and
convert at least a part of the relative depths included in the relative depth map into an absolute depth based on the marker depth.

2. The medical support device according to claim 1, wherein the processor is configured to generate an absolute depth map in which an absolute depth is assigned for each region of the surgical field image, based on the relative depth map and the marker depth.

3. The medical support device according to claim 2, wherein the processor is configured to perform control of displaying the surgical field image and the absolute depth map side by side or in a superimposed manner.

4. The medical support device according to any one of claims 1 to 3, wherein:
the surgical field includes a plurality of the markers,
the processor is configured to:
estimate the marker depth for each marker based on the surgical field image; and
convert at least a part of the relative depths included in the relative depth map into an absolute depth based on a plurality of the marker depths, and
optionally, the processor is configured to:
estimate validity of each marker depth; and
convert at least a part of the relative depths included in the relative depth map into an absolute depth based on a plurality of the marker depths and the validity.

5. The medical support device according to any one of claims 1 to 4, wherein:
the marker is assigned to a medical instrument, and
the processor is configured to:
acquire dimension information for each medical instrument; and
estimate the marker depth based on the surgical field image and the dimension information.

6. The medical support device according to any one of claims 1 to 5, wherein:
the marker is assigned to a medical instrument, and
the processor is configured to:
acquire presence information regarding a region in which each medical instrument is present in the surgical field image; and
estimate the marker depth based on the surgical field image and the presence information.

7. The medical support device according to any one of claims 1 to 6, wherein:
the processor is configured to:
acquire a plurality of the surgical field images that are temporally different;
for each of the surgical field images, generate the relative depth map, estimate the marker depth, and perform conversion into the absolute depth;
calculate an amount of variation in a corresponding absolute depth across the plurality of surgical field images; and
derive a single absolute depth based on the absolute depth converted for each of the surgical field images in a case where the amount of variation is equal to or less than a predetermined threshold value, and
optionally, the processor is configured to:
estimate validity of the absolute depth converted for each of the surgical field images; and
derive a single absolute depth based on the absolute depth converted for each of the surgical field images and the validity in a case where the amount of variation is equal to or less than the predetermined threshold value.

8. The medical support device according to any one of claims 1 to 7, wherein:
the processor is configured to:
extract a landmark from the surgical field image;
derive a landmark depth by converting a relative depth of a region that is included in the relative depth map and that corresponds to the landmark into an absolute depth based on the marker depth; and
convert a relative depth of at least a part of regions other than the region that is included in the relative depth map and that corresponds to the landmark into an absolute depth based on the landmark depth, and
optionally, the processor is configured to convert a relative depth of at least a part of regions other than the region that is included in the relative depth map and that corresponds to the landmark into an absolute depth based on the landmark depth in a case where the marker depth is not able to be estimated.

9. The medical support device according to any one of claims 1 to 8, wherein:
the processor is configured to:
estimate at least two marker depths based on the surgical field image;
derive scale information of a space shown in the surgical field image based on the at least two marker depths; and
perform control of displaying information corresponding to the scale information, and
optionally, the processor is configured to:
extract a superimposition object from the surgical field image; and
perform control of superimposing and displaying gradations related to the superimposition object on the surgical field image based on the absolute depth and the scale information.

10. The medical support device according to any one of claims 1 to 9, wherein the processor is configured to:
acquire a preoperative three-dimensional image of the surgical field;
extract a region of interest from the surgical field image;
identify a corresponding region that corresponds to a field of view of the surgical field image in the preoperative three-dimensional image based on the absolute depth and the region of interest; and
perform control of displaying the preoperative three-dimensional image based on the corresponding region.

11. The medical support device according to any one of claims 1 to 10, wherein:
the processor is configured to:
acquire a preoperative three-dimensional image of the surgical field;
extract a region of interest from the surgical field image;
register the region of interest with the preoperative three-dimensional image based on the absolute depth and the region of interest; and
perform control of superimposing and displaying the preoperative three-dimensional image on the surgical field image based on a result of the registration, and
for example, the processor is configured to:
identify at least one of a corresponding region that corresponds to a field of view of the surgical field image in the preoperative three-dimensional image or a non-rigid deformation parameter based on the result of the registration; and
perform control of superimposing and displaying the preoperative three-dimensional image on the surgical field image based on a result of the identification.

12. The medical support device according to any one of claims 1 to 11, wherein the processor is configured to:
acquire a preoperative three-dimensional image of the surgical field;
identify an adjacent blood vessel closest to a viewpoint of the surgical field image based on the surgical field image and the preoperative three-dimensional image; and
derive a distance between the adjacent blood vessel and a predetermined position based on the surgical field image and the absolute depth.

13. The medical support device according to any one of claims 1 to 12, wherein the processor is configured to:
acquire a preoperative three-dimensional image of the surgical field;
identify a target part inside an organ based on the preoperative three-dimensional image; and
derive a distance between the target part and a predetermined position based on the preoperative three-dimensional image, the surgical field image, and the absolute depth.

14. A medical support method executed by a computer, the medical support method comprising:
acquiring a surgical field image obtained by capturing a surgical field with a camera;
generating a relative depth map in which a relative depth is assigned for each region of the surgical field image, based on the surgical field image;
estimating a marker depth that is an absolute depth of a marker shown in the surgical field image, based on the surgical field image; and
converting at least a part of the relative depths included in the relative depth map into an absolute depth based on the marker depth.

15. A computer-readable storage medium storing a medical support program causing a computer to execute:
acquiring a surgical field image obtained by capturing a surgical field with a camera;
generating a relative depth map in which a relative depth is assigned for each region of the surgical field image, based on the surgical field image;
estimating a marker depth that is an absolute depth of a marker shown in the surgical field image, based on the surgical field image; and
converting at least a part of the relative depths included in the relative depth map into an absolute depth based on the marker depth.
